# EUROPEAN PATENT APPLICATION

(11) **EP 2 305 265 A1**
(43) Date of publication of application: **06.04.2011**
(21) Application number: 10175357.2
(22) Date of filing: 06.08.2004
(51) Int. Cl.: A61K 31/553, A61K 31/475, A61K 31/343, A61P 35/00, A61P 35/02

(54) **Combinations comprising staurosporines**

(30) Priority: 08.08.2003 US 493828 P
(62) Divisional of application: 04763879.6
(71) Applicant: Novartis AG, 4056 Basel (CH); Novartis Pharma GmbH, 1230 Wien (AT)
(72) Inventor: Atadja, Peter, Wisdom, Action, MA 01720 (US); Bhalla, Kapil, N., Tampa, FL 33611 (US); Cohen, Pamela, Sarah, Tenafly, NJ 07670 (US)
(74) Representative: Gruber, Markus

(57) **Abstract**

The present invention relates to a method of treating myelodysplastic syndromes, lymphomas and leukemias, and also solid tumors with a pharmaceutical combination of a FLT-3 kinase inhibitor and a histone deacetylase inhibitor (HDAI). It also relates to the use of a pharmaceutical combination of a histone deacetylase inhibitor and a FLT-3 kinase inhibitor for the treatment of the diseases or malignancies mentioned above and the use of such a pharmaceutical composition for the manufacture of a medicament for the treatment of these diseases or malignancies

## Description

The present invention relates to a method of treating myelodysplastic syndromes, lymphomas and leukemias, in particular acute myeloid leukemia (AML), and also solid tumors such as e.g. colorectal cancer (CRC) and non-small cell lung cancer (NSCLC) with a pharmaceutical combination of a FLT-3 kinase inhibitor and a histone deacetylase inhibitor (HDAI). It also relates to the use of a pharmaceutical combination of a histone deacetylase inhibitor and a FLT-3 kinase inhibitor for the treatment of the diseases or malignancies mentioned above and the use of such a pharmaceutical composition for the manufacture of a medicament for the treatment of these diseases or malignancies.

It has now surprisingly been found that FLT-3 kinase inhibitors in combination with histone deacetylase inhibitors (HDAI) possess therapeutic properties, which render them particularly useful for the treatment myelodysplastic syndromes, lymphomas and leukemias, in particular acute myeloid leukemia (AML), and also solid tumors such as e.g. colorectal cancer (CRC) and non-small cell lung cancer (NSCLC).

FLT-3 kinase inhibitors of particular interest for use in the inventive combination are staurosporine derivatives of formula 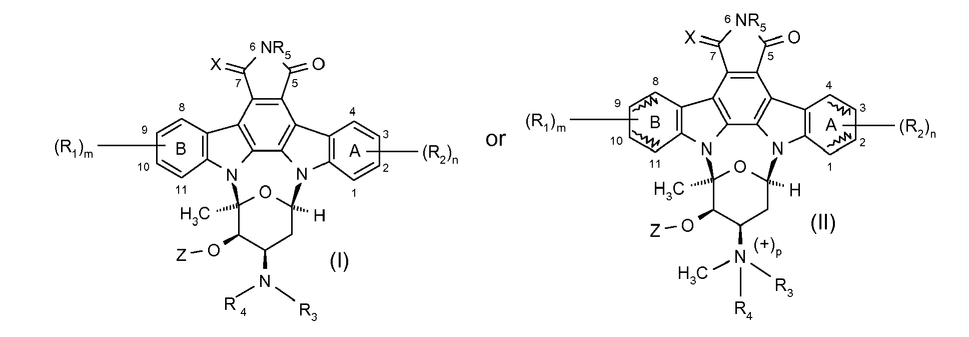 wherein (II) is the partially hydrogenated derivative of compound (I), or 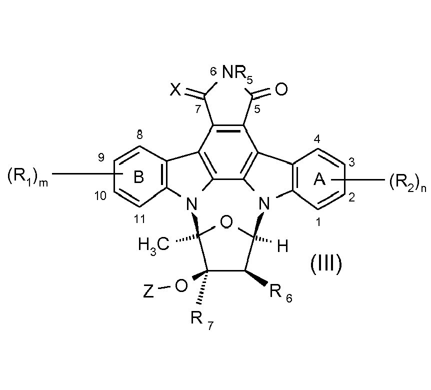 or 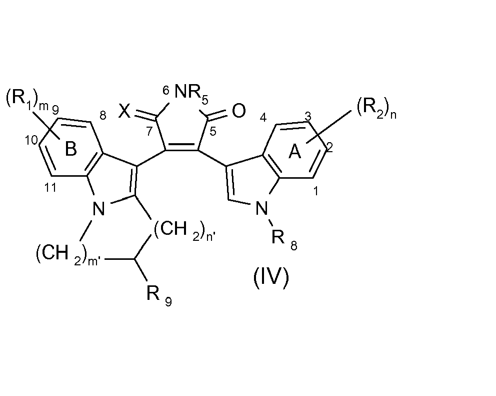 or 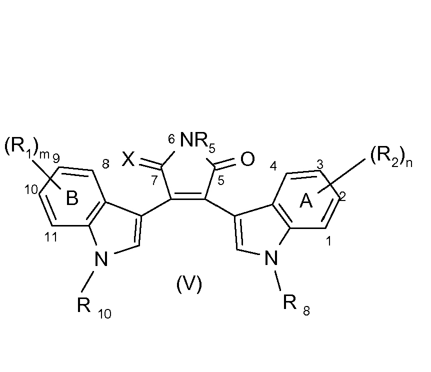 or 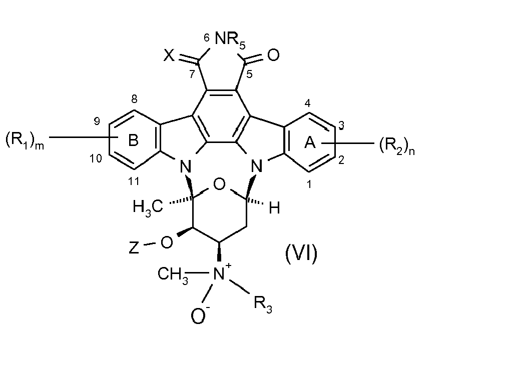 wherein R₁ and R₂, are, independently of one another, unsubstituted or substituted alkyl, hydrogen, halogen, hydroxy, etherified or esterified hydroxy, amino, mono- or disubstituted amino, cyano, nitro, mercapto, substituted mercapto, carboxy, esterified carboxy, carbamoyl, N-mono- or N,N-di-substituted carbamoyl, sulfo, substituted sulfonyl, aminosulfonyl or N-mono- or N,N-di-substituted aminosulfonyl;
n and m are, independently of one another, a number from and including 0 to and including 4;
n' and m' are, independently of one another, a number from and including 0 to and including 4;
R₃, R₄, R₈ and R₁₀ are, independently of one another, hydrogen, -O ⁻, acyl with up to 30 carbon atoms, an aliphatic, carbocyclic, or carbocyclic-aliphatic radical with up to 29 carbon atoms in each case, a heterocyclic or heterocyclic-aliphatic radical with up to 20 carbon atoms in each case, and in each case up to 9 heteroatoms, an acyl with up to 30 carbon atoms, wherein R₄ may also be absent;
or if R₃ is acyl with up to 30 carbon atoms, R₄ is not an acyl;
p is 0 if R₄ is absent, or is 1 if R₃ and R₄ are both present and in each case are one of the aforementioned radicals;
R₅ is hydrogen, an aliphatic, carbocyclic, or carbocyclic-aliphatic radical with up to 29 carbon atoms in each case, or a heterocyclic or heterocyclic-aliphatic radical with up to 20 carbon atoms in each case, and in each case up to 9 heteroatoms, or acyl with up to 30 carbon atoms;
R₇, R₆ and R₉ are acyl or -(lower alkyl) -acyl, unsubstituted or substituted alkyl, hydrogen, halogen, hydroxy, etherified or esterified hydroxy, amino, mono- or disubstituted amino, cyano, nitro, mercapto, substituted mercapto, carboxy,carbonyl, carbonyldioxy, esterified carboxy, carbamoyl, N-mono- or N,N-di-substituted carbamoyl, sulfo, substituted sulfonyl, aminosulfonyl or N-mono- or N,N-di-substituted aminosulfonyl;
X stands for 2 hydrogen atoms; for 1 hydrogen atom and hydroxy; for O; or for hydrogen and lower alkoxy;
Z stands for hydrogen or lower alkyl;
and either the two bonds characterised by wavy lines are absent in ring A and replaced by 4 hydrogen atoms, and the two wavy lines in ring B each, together with the respective parallel bond, signify a double bond;
or the two bonds characterised by wavy lines are absent in ring B and replaced by a total of 4 hydrogen atoms, and the two wavy lines in ring A each, together with the respective parallel bond, signify a double bond;
or both in ring A and in ring B all of the 4 wavy bonds are absent and are replaced by a total of 8 hydrogen atoms;
or a salt thereof, if at least one salt-forming group is present.

The general terms and definitions used hereinbefore and hereinafter preferably have the following meanings for the staurosporine derivatives:

The prefix "lower" indicates that the associated radical preferably has up to and including a maximum of 7 carbon atoms, especially up to and including a maximum of 4 carbon atoms.

Lower alkyl is especially methyl, ethyl, n-propyl, isopropyl, n-butyl, isobutyl, sec-butyl, or

tert-butyl, and also pentyl, hexyl, or heptyl.

Unsubstituted or substituted alkyl is preferably C₁-C₂₀alkyl, especially lower alkyl, typically methyl, ethyl, n-propyl, isopropyl, n-butyl, isobutyl, sec-butyl, or tert-butyl, which is unsubstituted or substituted especially by halogen, such as fluorine, chlorine, bromine, or iodine, C₆-C₁₄aryl, such as phenyl or naphthyl, hydroxy, etherified hydroxy, such as lower alkoxy, phenyl-lower alkoxy or phenyloxy, esterified hydroxy, such as lower alkanoyloxy or benzoyloxy, amino, mono- or disubstituted amino, such as lower alkylamino, lower alkanoylamino, phenyl-lower alkylamino, N,N-di-lower alkylamino, N,N-di-(phenyl-lower alkyl)amino, cyano, mercapto, substituted mercapto, such as lower alkylthio, carboxy, esterified carboxy, such as lower alkoxycarbonyl, carbamoyl, N-mono- or N,N-disubstituted carbamoyl, such as N-lower alkylcarbamoyl or N,N-di-lower alkylcarbamoyl, sulfo, substituted sulfo, such as lower alkanesulfonyl or lower alkoxysulfonyl, aminosulfonyl or N-mono- or N,N-disubstituted aminosulfonyl, such as N-lower alkylaminosulfonyl or N,N-di-lower alkylaminosulfonyl.

Halogen is preferably fluorine, chlorine, bromine, or iodine, especially fluorine or chlorine.

Etherified hydroxy is especially lower alkoxy, C₆-C₁₄aryloxy, such as phenyloxy, or C₆-C₁₄aryl-lower alkoxy, such as benzyloxy.

Esterified hydroxy is preferably lower alkanoyloxy or C₆-C₁₄arylcarbonyloxy, such as benzoyloxy.

Mono- or disubstituted amino is especially amino monosubstituted or disubstituted by lower alkyl, C₆-C₁₄aryl, C₆-C₁₄aryl-lower alkyl, lower alkanoyl, or C₆-C₁₂arylcarbonyl.

Substituted mercapto is especially lower alkylthio, C₆-C₁₄arylthio, C₆-C₁₄aryl-lower alkylthio, lower alkanoylthio, or C₆-C₁₄aryl-lower alkanoylthio.

Esterified carboxy is especially lower alkoxycarbonyl, C₆-C₁₄aryl-lower alkoxycarbonyl or C₆-C₁₄aryloxycarbonyl.

N-Mono- or N,N-disubstituted carbamoyl is especially carbamoyl N-monosubstituted or N,N-disubstituted by lower alkyl, C₆-C₁₄aryl or C₆-C₁₄aryl-lower alkyl.

Substituted sulfonyl is especially C₆-C₁₄arylsulfonyl, such as toluenesulfonyl, C₆-C₁₄aryl-lower alkanesulfonyl or lower alkanesulfonyl.

N-Mono- or N,N-disubstituted aminosulfonyl is especially aminosulfonyl N-monosubstituted or N,N-disubstituted by lower alkyl, C₆-C₁₄aryl or C₆-C₁₄aryl-lower alkyl.

C₆-C₁₄Aryl is an aryl radical with 6 to 14 carbon atoms in the ring system, such as phenyl, naphthyl, fluorenyl, or indenyl, which is unsubstituted or is substituted especially by halogen, such as fluorine, chlorine, bromine, or iodine, phenyl or naphthyl, hydroxy, lower alkoxy, phenyl-lower alkoxy, phenyloxy, lower alkanoyloxy, benzoyloxy, amino, lower alkylamino, lower alkanoylamino, phenyl-lower alkylamino, N,N-di-lower alkylamino, N,N-di-(phenyl-lower alkyl)amino, cyano, mercapto, lower alkylthio, carboxy, lower alkoxycarbonyl, carbamoyl, N-lower alkylcarbamoyl, N,N-di-lower alkylcarbamoyl, sulfo, lower alkanesulfonyl, lower alkoxysulfonyl, aminosulfonyl, N-lower alkylaminosulfonyl, or N,N-di-lower alkylaminosulfonyl.

The indices n and m are in each case preferably 1, 2 or especially 0. In general, compounds of formula I in which n and m are in each case 0 (zero) are especially preferred.

An aliphatic carbohydrate radical R₃, R₄, R₈ or R₁₀ with up to 29 carbon atoms, which is substituted by acyclic substituents and preferably has a maximum of 18, especially a maximum of 12, and as a rule not more than 7 carbon atoms, may be saturated or unsaturated and is especially an unsubstituted or a straight-chain or branched lower alkyl, lower alkenyl, lower alkadienyl, or lower alkinyl radical substituted by acyclic substituents. Lower alkyl is, for example, methyl, ethyl, n-propyl, isopropyl, n-butyl, isobutyl, sec-butyl or tert-butyl, and also n-pentyl, isopentyl, n-hexyl, isohexyl and n-heptyl; lower alkenyl is, for example, allyl, propenyl, isopropenyl, 2- or 3-methallyl and 2- or 3-butenyl; lower alkadienyl is, for example, 1-penta-2,4-dienyl; lower alkinyl is, for example, propargyl or 2-butinyl. In corresponding unsaturated radicals, the double bond is especially located in a position higher than the α-position in relation to the free valency. Substituents are especially the acyl radicals defined hereinbelow as substituents of R°, preferably free or esterified carboxy, such as carboxy or lower alkoxycarbonyl, cyano or di-lower alkylamino.

A carbocyclic or carbocyclic-aliphatic radical R₃, R₄, R₈ or R₁₀ with up to 29 carbon atoms in each case is especially an aromatic, a cycloaliphatic, a cycloaliphatic-aliphatic, or an aromatic-aliphatic radical which is either present in unsubstituted form or substituted by radicals referred to hereinbelow as substituents of R°. An aromatic radical (aryl radical) R₃ or R₄ is most especially a phenyl, also a naphthyl, such as 1- or 2-naphthyl, a biphenylyl, such as especially 4-biphenylyl, and also an anthryl, fluorenyl and azulenyl, as well as their aromatic analogues with one or more saturated rings, which is either present in unsubstituted form or substituted by radicals referred to hereinbelow as substituents of R°. Preferred aromatic-aliphatic radicals are aryl-lower alkyl- and aryl-lower alkenyl radicals, e.g. phenyl-lower alkyl or phenyl-lower alkenyl with a terminal phenyl radical, such as for example benzyl, phenethyl, 1-, 2-, or 3-phenylpropyl, diphenylmethyl (benzhydryl), trityl, and cinnamyl, and also 1- or 2-naphthylmethyl. Of aryl radicals carrying acyclic radicals, such as lower alkyl, special mention is made of o-, m- and p-tolyl and xylyl radicals with variously situated methyl radicals.

A cycloaliphatic radical R₃, R₄, R₈ or R₁₀ with up to 29 carbon atoms is especially a substituted or preferably unsubstituted mono-, bi-, or polycyclic cycloalkyl-, cycloalkenyl-, or cycloalkadienyl radical. Preference is for radicals with a maximum of 14, especially 12, ring-carbon atoms and 3- to 8-, preferably 5- to 7-, and most especially 6-member rings which can also carry one or more, for example two, aliphatic hydrocarbon radicals, for example those named above, especially the lower alkyl radicals, or other cycloaliphatic radicals as substituents. Preferred substituents are the acyclic substituents named hereinbelow for R°.

A cycloaliphatic-aliphatic radical R₃, R₄, R₈ or R₁₀ with up to 29 carbon atoms is a radical in which an acyclic radical, especially one with a maximum of 7, preferably a maximum of 4 carbon atoms, such as especially methyl, ethyl, and vinyl, carries one or more cycloaliphatic radicals as defined hereinabove. Special mention is made of cycloalkyl-lower alkyl radicals, as well as their analogues which are unsaturated in the ring and/or in the chain, but are non-aromatic, and which carry the ring at the terminal carbon atom of the chain. Preferred substituents are the acyclic substituents named herein below for R°.

Heterocyclic radicals R₃, R₄, R₈ or R₁₀ with up to 20 carbon atoms each and up to 9 heteroatoms each are especially monocyclic, but also bi- or polycyclic, aza-, thia-, oxa-, thiaza-, oxaza-, diaza-, triaza-, or tetrazacyclic radicals of an aromatic character, as well as corresponding heterocyclic radicals of this type which are partly or most especially wholly saturated, these radicals - if need be - possibly carrying further acyclic, carbocyclic, or heterocyclic radicals and/or possibly mono-, di-, or polysubstituted by functional groups, preferably those named hereinabove as substituents of aliphatic hydrocarbon radicals. Most especially they are unsubstituted or substituted monocyclic radicals with a nitrogen, oxygen, or sulfur atom, such as 2-aziridinyl, and especially aromatic radicals of this type, such as pyrryl, for example 2-pyrryl or 3-pyrryl, pyridyl, for example 2-, 3-, or 4-pyridyl, and also thienyl, for example 2- or 3-thienyl, or furyl, for example 2-furyl; analogous bicyclic radicals with an oxygen, sulfur, or nitrogen atom are, for example, indolyl, typically 2- or 3-indolyl, quinolyl, typically 2- or 4-quinolyl, isoquinolyl, typically 3- or 5-isoquinolyl, benzofuranyl, typically 2-benzofuranyl, chromenyl, typically 3-chromenyl, or benzothienyl, typically 2- or 3-benzothienyl; preferred monocyclic and bicyclic radicals with several heteroatoms are, for example, imidazolyl, typically 2- or 4-imidazolyl, pyrimidinyl, typically 2-or 4-pyrimidinyl, oxazolyl, typically 2-oxazolyl, isoxazolyl, typically 3-isoxazolyl, or thiazolyl, typically 2-thiazolyl, and benzimidazolyl, typically 2-benzimidazolyl, benzoxazolyl, typically 2-benzoxazolyl, or quinazolyl, typically 2-quinazolinyl. Appropriate partially or, especially, completely saturated analogous radicals may also be considered, such as 2-tetrahydrofuryl, 2- or 3-pyrrolidinyl, 2-, 3-, or 4-piperidyl, and also 2-or 3-morpholinyl, 2- or 3-thiomorpholinyl, 2-piperazinyl and N-mono- or N,N'-bis-lower alkyl-2-piperazinyl radicals. These radicals may also carry one or more acyclic, carbocyclic, or heterocyclic radicals, especially those mentioned hereinabove. The free valency of the heterocyclic radicals R₃ or R₄ must emanate from one of their carbon atoms. Heterocyclyl may be unsubstituted or substituted by one or more, preferably one or two, of the substituents named hereinbelow for R°. Heterocyclic-aliphatic radicals R₃, R₄, R₈ or R₁₀ especially lower alkyl radicals, especially with a maximum of 7, preferably a maximum of 4 carbon atoms, for example those named hereinabove, which carry one, two, or more heterocyclic radicals, for example those named in the preceding paragraph, the heterocyclic ring possibly being linked to the aliphatic chain also by one of its nitrogen atoms. A preferred heterocyclic-aliphatic radical R₁ is, for example, imidazol-1-ylmethyl, 4-methylpiperazin-1-ylmethyl, piperazin-1-ylmethyl, 2-(morpholin-4-yl)ethyl and also pyrid-3-ylmethyl. Heterocyclyl may be unsubstituted or substituted by one or more, preferably one or two, of the substituents named hereinbelow for R°.

A heteroaliphatic radical R₃, R₄, R₈ or R₁₀ with up to 20 carbon atoms each and up to 10 heteroatoms each is an aliphatic radical which, instead of one, two, or more carbon atoms, contains identical or different heteroatoms, such as especially oxygen, sulfur, and nitrogen. An especially preferred arrangement of a heteroaliphatic radical R₁ takes the form of oxa-alkyl radicals in which one or more carbon atoms are replaced in a preferably linear alkyl by oxygen atoms preferably separated from one another by several (especially 2) carbon atoms so that they form a repeating group, if need be multi-repeating group (O-CH₂-CH₂-)_{q}, wherein q = 1 to 7.

Especially preferred as R₃, R₄, R₈ or R₁₀, apart from acyl, is lower alkyl, particlularly methyl or ethyl; lower alkoxycarbonyl-lower alkyl, especially methoxycarbonylmethyl or 2-(tert-butoxycarbonyl)ethyl; carboxy-lower alkyl, especially carboxymethyl or 2-carboxyethyl; or cyano-lower alkyl, especially 2-cyanoethyl.

An acyl radical R₃, R₄, R₆, R₇, R₈, R₉, or R₁₀with up to 30 carbon atoms derives from a carboxylic acid, functionally modified if need be, an organic sulfonic acid, or a phosphoric acid, such as pyro- or orthophosphoric acid, esterified if need be.

An acyl designated Ac¹ and derived from a carboxylic acid, functionally modified if need be, is especially one of the subformula Y-C(=W)-, wherein W is oxygen, sulfur, or imino and Y is hydrogen, hydrocarbyl R° with up to 29 carbon atoms, hydrocarbyloxy R°-O-, an amino group or a substituted amino group, especially one of the formula R°HN- or R°R°N- (wherein the R° radicals may be identical or different from one another).

The hydrocarbyl (hydrocarbon radical) R° is an acyclic (aliphatic), carbocyclic, or carbocyclic-acyclic hydrocarbon radical, with up to 29 carbon atoms each, especially up to 18, and preferably up to 12 carbon atoms, and is saturated or unsaturated, unsubstituted or substituted. Instead of one, two, or more carbon atoms, it may contain identical or different heteroatoms, such as especially oxygen, sulfur, and nitrogen in the acyclic and/or cyclic part; in the latter case, it is described as a heterocyclic radical (heterocyclyl radical) or a heterocyclic-acyclic radical.

Unsaturated radicals are those, which contain one or more, especially conjugated and/or isolated, multiple bonds (double or triple bonds). The term cyclic radicals includes also aromatic and non-aromatic radicals with conjugated double bonds, for example those wherein at least one 6-member carbocyclic or a 5- to 8-member heterocyclic ring contains the maximum number of non-cumulative double bonds. Carbocyclic radicals, wherein at least one ring is present as a 6-member aromatic ring (i.e. a benzene ring), are defined as aryl radicals.

An acyclic unsubstituted hydrocarbon radical R° is especially a straight-chained or branched lower alkyl-, lower alkenyl-, lower alkadienyl-, or lower alkinyl radical. Lower alkyl R° is, for example, methyl, ethyl, n-propyl, isopropyl, n-butyl, isobutyl, sec-butyl or tert-butyl, and also n-pentyl, isopentyl, n-hexyl, isohexyl and n-heptyl; lower alkenyl is, for example, allyl, propenyl, isopropenyl, 2- or 3-methallyl and 2- or 3-butenyl; lower alkadienyl is, for example, 1-penta-2,4-dienyl; lower alkinyl is, for example, propargyl or 2-butinyl. In corresponding unsaturated radicals, the double bond is especially located in a position higher than the α-position in relation to the free valency.

A carbocyclic hydrocarbon radical R° is especially a mono-, bi-, or polycyclic cycloalkyl-, cycloalkenyl-, or cycloalkadienyl radical, or a corresponding aryl radical. Preference is for radicals with a maximum of 14, especially 12, ring-carbon atoms and 3- to 8-, preferably 5-to 7-, and most especially 6-member rings which can also carry one or more, for example two, acyclic radicals, for example those named above, especially the lower alkyl radicals, or other carbocyclic radicals. Carbocyclic-acyclic radicals are those in which an acyclic radical, especially one with a maximum of 7, preferably a maximum of 4 carbon atoms, such as especially methyl, ethyl and vinyl, carries one or more carbocyclic, if need be aromatic radicals of the above definition. Special mention is made of cycloalkyl-lower and aryl-lower alkyl radicals, as well as their analogues which are unsaturated in the ring and/or chain, and which carry the ring at the terminal carbon atom of the chain.

Cycloalkyl R° has most especially from 3 up to and including 10 carbon atoms and is, for example, cyclopropyl, cyclobutyl, cyclopentyl, cyclohexyl, cycloheptyl, and cyclooctyl, as well as bicyclo[2,2,2]octyl, 2-bicyclo[2,2,1]heptyl, and adamantyl, which may also be substituted by 1, 2, or more, for example lower, alkyl radicals, especially methyl radicals; cycloalkenyl is for example one of the monocyclic cycloalkyl radicals already named which carries a double bond in the 1-, 2-, or 3 position. Cycloalkyl-lower alkyl or -lower alkenyl is for example a -methyl, -1- or -2-ethyl, -1- or -2-vinyl, -1-, -2-, or -3-propyl or -allyl substituted by one of the above-named cycloalkyl radicals, those substituted at the end of the linear chain being preferred.

An aryl radical R⁰ is most especially a phenyl, also a naphthyl, such as 1- or 2-naphthyl, a biphenylyl, such as especially 4-biphenylyl, and also an anthryl, fluorenyl and azulenyl, as well as their aromatic analogues with one or more saturated rings. Preferred aryl-lower alkyl and -lower alkenyl radicals are, for example, phenyl-lower alkyl or phenyl-lower alkenyl with a terminal phenyl radical, such as for example benzyl, phenethyl, 1-, 2-, or 3-phenylpropyl, diphenylmethyl (benzhydryl), trityl, and cinnamyl, and also 1- or 2-naphthylmethyl. Aryl may be unsubstituted or substituted.

Heterocyclic radicals, including heterocyclic-acyclic radicals, are especially monocyclic, but also bi- or polycyclic, aza-, thia-, oxa-, thiaza-, oxaza-, diaza-, triaza-, or tetrazacyclic radicals of an aromatic character, as well as corresponding heterocyclic radicals of this type which are partly or most especially wholly saturated; if need be, for example as in the case of the above-mentioned carbocyclic or aryl radicals, these radicals may carry further acyclic, carbocyclic, or heterocyclic radicals and/or may be mono-, di-, or polysubstituted by functional groups. The acyclic part in heterocyclic-acyclic radicals has for example the meaning indicated for the corresponding carbocyclic-acyclic radicals. Most especially they are unsubstituted or substituted monocyclic radicals with a nitrogen, oxygen, or sulfur atom, such as 2-aziridinyl, and especially aromatic radicals of this type, such as pyrrolyl, for example 2-pyrrolyl or 3-pyrrolyl, pyridyl, for example 2-, 3-, or 4-pyridyl, and also thienyl, for example 2- or 3-thienyl, or furyl, for example 2-furyl; analogous bicyclic radicals with an oxygen, sulfur, or nitrogen atom are, for example, indolyl, typically 2- or 3-indolyl, quinolyl, typically 2- or 4-quinolyl, isoquinolyl, typically 3- or 5-isoquinolyl, benzofuranyl, typically 2-benzofuranyl, chromenyl, typically 3-chromenyl, or benzothienyl, typically 2- or 3-benzothienyl; preferred monocyclic and bicyclic radicals with several heteroatoms are, for example, imidazolyl, typically 2-imidazolyl, pyrimidinyl, typically 2-or 4-pyrimidinyl, oxazolyl, typically 2-oxazolyl, isoxazolyl, typically 3-isoxazolyl, or thiazolyl, typically 2-thiazolyl, and benzimidazolyl, typically 2-benzimidazolyl, benzoxazolyl, typically 2-benzoxazolyl, or quinazolyl, typically 2-quinazolinyl. Appropriate partially or, especially, completely saturated analogous radicals may also be considered, such as 2-tetrahydrofuryl, 4-tetrahydrofuryl, 2-or 3-pyrrolidyl, 2-, 3-, or 4-piperidyl, and also 2-or 3-morpholinyl, 2- or 3-thiomorpholinyl, 2-piperazinyl, and N,N'-bis-lower alkyl-2-piperazinyl radicals. These radicals may also carry one or more acyclic, carbocyclic, or heterocyclic radicals, especially those mentioned hereinabove. Heterocyclic-acyclic radicals are especially derived from acyclic radicals with a maximum of 7, preferably a maximum of 4 carbon atoms, for example those named hereinabove, and may carry one, two, or more heterocyclic radicals, for example those named hereinabove, the ring possibly being linked to the aliphatic chain also by one of its nitrogen atoms.

As already mentioned, a hydrocarbyl (including a heterocyclyl) may be substituted by one, two, or more identical or different substituents (functional groups); one or more of the following substituents may be considered: lower alkyl; free, etherified and esterified hydroxyl groups; carboxy groups and esterified carboxy groups; mercapto- and lower alkylthio- and, if need be, substituted phenylthio groups; halogen atoms, typically chlorine and fluorine, but also bromine and iodine; *halogen-lower alkyl groups;* oxo groups which are present in the form of formyl (i.e. aldehydo) and keto groups, also as corresponding acetals or ketals; azido groups; nitro groups; cyano groups; primary, secondary and preferably tertiary amino groups, amino-lower alkyl, mono- or disubstituted amino-lower alkyl, primary or secondary amino groups protected by conventional protecting groups (especially lower alkoxycarbonyl, typically tert-butoxycarbonyl) lower alkylenedioxy, and also free or functionally modified sulfo groups, typically sulfamoyl or sulfo groups present in free form or as salts. The hydrocarbyl radical may also carry carbamoyl, ureido, or guanidino groups, which are free or which carry one or two substituents, and cyano groups. The above use of the word "groups" is taken to imply also an individual group.

Halogen-lower alkyl contains preferably 1 to 3 halogen atoms; preferred is trifluoromethyl or chloromethyl.

An etherified hydroxyl group present in the hydrocarbyl as substituent is, for example, a lower alkoxy group, typically the methoxy-, ethoxy-, propoxy-, isopropoxy-, butoxy-, and tert-butoxy group, which may also be substituted, especially by (i) heterocyclyl, whereby heterocyclyl can have preferably 4 to 12 ring atoms, may be unsaturated, or partially or wholly saturated, is mono- or bicyclic, and may contain up to three heteroatoms selected from nitrogen, oxygen, and sulfur, and is most especially pyrrolyl, for example 2-pyrrolyl or 3-pyrrolyl, pyridyl, for example 2-, 3- or 4-pyridyl, and also thienyl, for example 2- or 3-thienyl, or furyl, for example 2-furyl, indolyl, typically 2- or 3-indolyl, quinolyl, typically 2- or 4-quinolyl, isoquinolyl, typically 3- or 5-isoquinolyl, benzofuranyl, typically 2-benzofuranyl, chromenyl, typically 3-chromenyl, benzothienyl, typically 2- or 3-benzothienyl; imidazolyl, typically 1- or 2-imidazolyl, pyrimidinyl, typically 2-or 4-pyrimidinyl, oxazolyl, typically 2-oxazolyl, isoxazolyl, typically 3-isoxazolyl, thiazolyl, typically 2-thiazolyl, benzimidazolyl, typically 2-benzimidazolyl, benzoxazolyl, typically 2-benzoxazolyl, quinazolyl, typically 2-quinazolinyl, 2-tetrahydrofuryl, 4-tetrahydrofuryl, 2- or 4-tetrahydropyranyl, 1-, 2- or 3-pyrrolidyl, 1-, 2-, 3-, or 4-piperidyl, 1-, 2-or 3-morpholinyl, 2- or 3-thiomorpholinyl, 2-piperazinyl or N,N'-bis-lower alkyl-2-piperazinyl; and also (ii) by halogen atoms, for example mono-, di-, or polysubstituted especially in the 2-position, as in the 2,2,2-trichloroethoxy, 2-chloroethoxy, or 2-iodoethoxy radical, or (iii) by hydroxy or (iv) lower alkoxy radicals, each preferably monosubstituted, especially in the 2-position, as in the 2-methoxyethoxy radical. Such etherified hydroxyl groups are also unsubstituted or substituted phenoxy radicals and phenyl-lower alkoxy radicals, such as especially benzyloxy, benzhydryloxy, and triphenylmethoxy (trityloxy), as well as heterocyclyloxy radicals, wherein heterocyclyl can have preferably 4 to 12 ring atoms, may be unsaturated, or partially or wholly saturated, is mono- or bicyclic, and may contain up to three heteroatoms selected from nitrogen, oxygen, and sulfur, and is most especially pyrrolyl, for example 2-pyrrolyl or 3-pyrrolyl, pyridyl, for example 2-, 3- or 4-pyridyl, and also thienyl, for example 2- or 3-thienyl, or furyl, for example 2-furyl, indolyl, typically 2- or 3-indolyl, quinolyl, typically 2- or 4-quinolyl, isoquinolyl, typically 3- or 5-isoquinolyl, benzofuranyl, typically 2-benzofuranyl, chromenyl, typically 3-chromenyl, benzothienyl, typically 2- or 3-benzothienyl; imidazolyl, typically 1- or 2-imidazolyl, pyrimidinyl, typically 2- or 4-pyrimidinyl, oxazolyl, typically 2-oxazolyl, isoxazolyl, typically 3-isoxazolyl, thiazolyl, typically 2-thiazolyl, benzimidazolyl, typically 2-benzimidazolyl, benzoxazolyl, typically 2-benzoxazolyl, quinazolyl, typically 2-quinazolinyl, 2-tetrahydrofuryl, 4-tetrahydrofuryl, 2- or 4-tetrahydropyranyl, 1-, 2- or 3-pyrrolidyl, 1-, 2-, 3-, or 4-piperidyl, 1-, 2-or 3-morpholinyl, 2- or 3-thiomorpholinyl, 2-piperazinyl or N,N'-bis-lower alkyl-2-piperazinyl; such as especially 2- or 4-tetrahydropyranyloxy.

Etherified hydroxyl groups in this context are taken to include silylated hydroxyl groups, typically for example tri-lower alkylsilyloxy, typically trimethylsilyloxy and dimethyl-tert-butylsilyloxy, or phenyldi-lower alkylsilyloxy and lower alkyl-diphenylsilyloxy.

An esterified hydroxyl group present in the hydrocarbyl as a substituent is, for example, lower alkanoyloxy.

A carboxyl group present in the hydrocarbyl as a substituent is one in which the hydrogen atom is replaced by one of the hydrocarbyl radicals characterised hereinabove, preferably a lower alkyl- or phenyl-lower alkyl radical; an example of an esterified carboxyl group is lower alkoxycarbonyl or phenyl-lower alkoxycarbonyl substituted if need be in the phenyl part, especially the methoxy, ethoxy, tert-butoxy, and benzyloxycarbonyl group, as well as a lactonised carboxyl group.

A primary amino group -NH₂ as substituent of the hydrocarbyls may also be present in a form protected by a conventional protecting group. A secondary amino group carries, instead of one of the two hydrogen atoms, a hydrocarbyl radical, preferably an unsubstituted one, typically one of the above-named, especially lower alkyl, and may also be present in protected form.

A tertiary amino group present in the hydrocarbyl as substituent carries 2 different or, preferably, identical hydrocarbyl radicals (including the heterocyclic radicals), such as the unsubstituted hydrocarbyl radicals characterised hereinabove, especially lower alkyl.

A preferred amino group is one with the formula R₁₁(R₁₂)N-, wherein R₁₁ and R₁₂ are independently in each case hydrogen, unsubstituted acyclic C₁-C₇-hydrocarbyl (such as especially C₁-C₄alkyl or C₂-C₄alkenyl) or monocyclic aryl, aralkyl, or aralkenyl, substituted if necessary by C₁-C₄-alkyl, C₁-C₄-alkoxy, halogen, and/or nitro, and having a maximum of 10 carbon atoms, where the carbon-containing radicals may be interlinked through a carbon-carbon bond or an oxygen atom, a sulfur atom, or a nitrogen atom substituted if necessary by hydrocarbyl. In such a case, they form a nitrogen-containing heterocyclic ring with the nitrogen atom of the amino group. The following are examples of especially preferred disubstituted amino groups: di-lower alkylamino, typically dimethylamino or diethylamino, pyrrolidino, imidazol-1-yl, piperidino, piperazino, 4-lower alkylpiperazino, morpholino, thiomorpholino and piperazino or 4-methylpiperazino, as well as diphenylamino and dibenzylamino substituted if need be, especially in the phenyl part, for example by lower-alkyl, lower-alkoxy, halogen, and/or nitro; of the protected groups, especially lower alkoxycarbonylamino, typically tert-butoxycarbonylamino, phenyl-lower alkoxycarbonylamino, typically 4-methoxybenzyloxycarbonylamino, and 9-fluorenylmethoxycarbonylamino.

Amino-lower alkyl is most especially substituted in the 1-position of the lower alkyl chain by amino and is especially aminomethyl.

Mono- or disubstituted amino-lower alkyl is amino-lower alkyl substituted by one or two radicals, wherein amino-lower alkyl is most especially substituted by amino in the 1-position of the lower alkyl chain and is especially aminomethyl; the amino substituents here are preferably (if 2 substituents are present in the respective amino group independently of one another) from the group comprising lower alkyl, such as especially methyl, ethyl or n-propyl, hydroxy-lower alkyl, typically 2-hydroxyethyl, C₃-C₈cycloalkyl, especially cyclohexyl, amino-lower alkyl, typically 3-aminopropyl or 4-aminobutyl, N-mono- or N,N-di(lower alkyl)-amino-lower alkyl, typically 3-(N,N-dimethylamino)propyl, amino, N-mono- or N,N-di-lower alkylamino and N-mono- or N,N-di-(hydroxy-lower alkyl)amino.

Disubstituted amino-lower alkyl is also a 5 or 6-membered, saturated or unsaturated heterocyclyl bonded to lower alkyl via a nitrogen atom (preferably in the 1-position) and having 0 to 2, especially 0 or 1, other heteroatoms selected from oxygen, nitrogen, and sulfur, which is unsubstituted or substituted, especially by one or two radicals from the group comprising lower alkyl, typically methyl, and also oxo. Preferred here is pyrrolidino (1-pyrrolidinyl), piperidino (1-piperidinyl), piperazino (1-piperazinyl), 4-lower alkylpiperazino, typically 4-methylpiperazino, imidazolino (1-imidazolyl), morpholino (4-morpholinyl), or also thiomorpholino, S-oxo-thiomorpholino, or S,S-dioxothiomorpholino.

Lower alkylenedioxy is especially methylenedioxy.

A carbamoyl group carrying one or two substituents is especially aminocarbonyl (carbamoyl) which is substitiuted by one or two radicals at the nitrogen; the amino substituents here are preferably (if 2 substituents are present in the respective amino group independently of one another) from the group comprising lower alkyl, such as especially methyl, ethyl or n-propyl, hydroxy-lower alkyl, typically 2-hydroxyethyl, C₃-C₈cycloalkyl, especially cyclohexyl, amino-lower alkyl, typically 3-aminopropyl or 4-aminobutyl, N-mono- or N,N-di(lower alkyl)-amino-lower alkyl, typically 3-(N,N-dimethylamino)propyl, amino, N-mono- or N,N-di-lower alkylamino and N-mono- or N,N-di-(hydroxy-lower alkyl)amino; disubstituted amino in aminocarbamoyl is also a 5 or 6-membered, saturated or unsaturated heterocyclyl with a bonding nitrogen atom and 0 to 2, especially 0 or 1, other heteroatoms selected from oxygen, nitrogen, and sulfur, which is unsubstituted or substituted, especially by one or two radicals from the group comprising lower alkyl, typically methyl, and also oxo. Preferred here is pyrrolidino (1-pyrrolidinyl), piperidino (1-piperidinyl), piperazino (1-piperazinyl), 4-lower alkylpiperazino, typically 4-methylpiperazino, imidazolino (1-imidazolyl), morpholino (4-morpholinyl), or also thiomorpholino, S-oxo-thiomorpholino, or S,S-dioxothiomorpholino.

An acyl derived from an organic sulfonic acid, which is designated Ac², is especially one with the subformula R°-SO₂-, wherein R° is a hydrocarbyl as defined above in the general and specific meanings, the latter also being generally preferred here. Especially preferred is lower alkylphenylsulfonyl, especially 4-toluenesulfonyl.

An acyl derived from a phosphoric acid, esterified if necessary, which is designated Ac³, is especially one with the subformula R°O(R°O)P(=O)-, wherein the radicals R° are, independently of one another, as defined in the general and specific meanings indicated above.

Reduced data on substituents given hereinbefore and hereinafter are considered to be preferences.

Preferred compounds according to the invention are, for example, those wherein R⁰ has the following preferred meanings: lower alkyl, especially methyl or ethyl, amino-lower alkyl, wherein the amino group is unprotected or is protected by a conventional amino protecting group - especially by lower alkoxycarbonyl, typically tert-lower alkoxycarbonyl, for example tert-butoxycarbonyl - e.g. aminomethyl, R,S-, R- or preferably S-1-aminoethyl, tert-butoxycarbonylaminomethyl or R,S-, R-, or preferably S-1-(tert-butoxycarbonylamino)ethyl, carboxy-lower alkyl, typically 2-carboxyethyl, lower alkoxycarbonyl-lower alkyl, typically 2-(tert-butoxycarbonyl)ethyl, cyano-lower alkyl, typically 2-cyanoethyl, tetrahydropyranyloxy-lower alkyl, typically 4-(tetrahydropyranyl)-oxymethyl, morpholino-lower alkyl, typically 2-(morpholino)ethyl, phenyl, lower alkylphenyl, typically 4-methylphenyl, lower alkoxyphenyl, typically 4-methoxyphenyl, imidazolyl-lower alkoxyphenyl, typically 4-[2-(imidazol-1-yl)ethyl)oyxphenyl, carboxyphenyl, typically 4-carboxyphenyl, lower alkoxycarbonylphenyl, typically 4-ethoxycarbonylphenyl or 4-methoxyphenyl, halogen-lower alkylphenyl, typically 4-chloromethylphenyl, pyrrolidinophenyl, typically 4-pyrrolidinophenyl, imidazol-1-ylphenyl, typically 4-(imidazolyl-1-yl)phenyl, piperazinophenyl, typically 4-piperazinophenyl, (4-lower alkylpiperazino)phenyl, typically 4-(4-methylpiperazino)phenyl, morpholinophenyl, typically 4-morpholinophenyl, pyrrolidino-lower alkylphenyl, typically 4-pyrrolidinomethylphenyl, imidazol-1-yl-lower alkylphenyl, typically 4-(imidazolyl-1-ylmethyl)phenyl, piperazino-lower alkylphenyl, typically 4-piperazinomethylphenyl, (4-lower alkylpiperazinomethyl)-phenyl, typically 4-(4-methylpiperazinomethyl)phenyl, morpholino-lower alkylphenyl, typically 4-morpholinomethylphenyl, piperazinocarbonylphenyl, typically 4-piperazinocarbonylphenyl, or (4-lower alkyl-piperazino)phenyl, typically 4-(4-methylpiperazino)phenyl.

Preferred acyl radicals Ac¹ are acyl radicals of a carboxylic acid which are characterised by the subformula R°-CO-, wherein R° has one of the above general and preferred meanings of the hydrocarbyl radical R°. Especially preferred radicals R° here are lower alkyl, especially methyl or ethyl, amino-lower alkyl, wherein the amino group is unprotected or protected by a conventional amino protecting group, especially by lower alkoxycarbonyl, typically tert-lower alkoxycarbonyl, for example tert-butoxycarbonyl, e.g. aminomethyl, R,S-, R-, or preferably S-1-aminoethyl, tert-butoxycarbonylaminomethyl or R,S-, R-, or preferably S-1-(tert-butoxycarbonylamino)ethyl, carboxy-lower alkyl, typically 2-carboxyethyl, lower alkoxycarbonyl-lower alkyl, typically 2-(tert-butoxycarbonyl)ethyl, tetrahydropyranyloxy-lower alkyl, typically 4-(tetrahydropyranyl)oxymethyl, phenyl, imidazolyl-lower alkoxyphenyl, typically 4-[2-(imidazol-1-yl)ethyl]oyxphenyl, carboxyphenyl, typically 4-carboxyphenyl, lower alkoxycarbonylphenyl, typically 4-ethoxycarbonylphenyl, halogen-lower alkylphenyl, typically 4-chloromethylphenyl, imidazol-1-ylphenyl, typically 4-(imidazolyl-1-yl)phenyl, pyrrolidino-lower alkylphenyl, typically 4-pyrrolidinomethylphenyl, piperazino-lower alkylphenyl, typically 4-piperazinomethylphenyl, (4-lower alkylpiperazinomethyl)phenyl, typically 4-(4-methyl-piperazinomethyl)phenyl, morpholino-lower alkylphenyl, typically 4-morpholinomethylphenyl, piperazinocarbonylphenyl, typically 4-piperazinocarbonylphenyl, or (4-lower alkylpiperazino)phenyl, typically 4-(4-methylpiperazino)phenyl.

A further preferred Acyl Ac¹ is derived from monoesters of carbonic acid and is characterised by the subformula R°-O-CO-. The lower alkyl radicals, especially tert-butyl, are especially preferred hydrocarbyl radicals R° in these derivatives.

Another preferred Acyl Ac¹ is derived from amides of carbonic acid (or also thiocarbonic acid) and is characterised by the formula R°HN-C(=W)- or R°R°N-C(=W)-, wherein the radicals R° are, independently of one another, as defined above and W is sulfur and especially oxygen. In particular, compounds are preferred wherein Ac¹ is a radical of formula R°HN-C(=W)-, wherein W is oxygen and R° has one of the following preferred meanings: morpholino-lower alkyl, typically 2-morpholinoethyl, phenyl, lower alkoxyphenyl, typically 4-methoxyphenyl or 4-ethoxyphenyl, carboxyphenyl, typically 4-carboxyphenyl, or lower alkoxycarbonylphenyl, typically 4-ethoxycarbonylphenyl.

A preferred acyl Ac² of subformula R°-SO₂-, wherein R° is a hydrocarbyl as defined in the above general and specific meanings, is lower alkylphenylsulfonyl, typically 4-toluenesulfonyl.

If p is 0, the nitrogen atom bonding R₃ is uncharged. If p is 1, then R₄ must also be present, and the nitrogen atom bonding R₃ and R₄ (quaternary nitrogen) is then positively charged.

The definitions for an aliphatic, carbocyclic, or carbocyclic-aliphatic radical with up to 29 carbon atoms each, or for a heterocyclic or heterocyclic-aliphatic radical with up to 20 carbon atoms each and up to 9 heteroatoms each, or acyl with up to 30 carbon atoms each, preferably match the definitions given for the corresponding radicals R₃ and R₄. Especially preferred is R₅ lower alkyl, especially methyl, or most especially hydrogen.

Z is especially lower alkyl, most especially methyl or hydrogen.

If the two bonds indicated by wavy lines are missing in ring A, then no double bonds (tetra-hydrogenated derivatives) are present between the carbon atoms characterised in formula I by the numbers 1, 2, 3, and 4, but only single bonds, whereas ring B is aromatic (double bonds between the carbon atoms characterised in formula I by 8 and 9 and those characterised by 10 and 11). If the two bonds indicated by wavy lines are missing in ring B, then no double bonds (tetra-hydrogenated derivatives) are present between the carbon atoms characterised in formula I by the numbers 8, 9, 10, and 11, but only single bonds, whereas ring A is aromatic (double bonds between the carbon atoms characterised in formula I by 1 and 2 and those characterised by 3 and 4). If the total of four bonds indicated by wavy lines are missing in rings A and B, and are replaced by a total of 8 hydrogen atoms, then no double bonds (octa-hydrogenated derivatives) are present between the carbon atoms numbered 1, 2, 3, 4, 8, 9, 10, and 11 in formula I, but only single bonds.

By their nature, the compounds of the invention may also be present in the form of pharmaceutically, i.e. physiologically, acceptable salts, provided they contain salt-forming groups. For isolation and purification, pharmaceutically unacceptable salts may also be used. For therapeutic use, only pharmaceutically acceptable salts are used, and these salts are preferred.

Thus, compounds of formula I having free acid groups, for example a free sulfo, phosphoryl or carboxyl group, may exist as a salt, preferably as a physiologically acceptable salt with a salt-forming basic component. These may be primarily metal or ammonium salts, such as alkali metal or alkaline earth metal salts, for example sodium, potassium, magnesium or calcium salts, or ammonium salts with ammonia or suitable organic amines, especially tertiary monoamines and heterocyclic bases, for example triethylamine, tri-(2-hydroxyethyl)-amine, N-ethylpiperidine or N,N'-dimethylpiperazine.

Compounds of the invention having a basic character may also exist as addition salts, especially as acid addition salts with inorganic and organic acids, but also as quaternary salts. Thus, for example, compounds which have a basic group, such as an amino group, as a substituent may form acid addition salts with common acids. Suitable acids are, for example, hydrohalic acids, e.g. hydrochloric and hydrobromic acid, sulfuric acid, phosphoric acid, nitric acid or perchloric acid, or aliphatic, alicyclic, aromatic or heterocyclic carboxylic or sulfonic acids, such as formic, acetic, propionic, succinic, glycolic, lactic, malic, tartaric, citric, fumaric, maleic, hydroxymaleic, oxalic, pyruvic, phenylacetic, benzoic, p-aminobenzoic, anthranilic, p-hydroxybenzoic, salicylic, p-aminosalicylic acid, pamoic acid, methanesulfonic, ethanesulfonic, hydroxyethanesulfonic, ethylenedisulfonic, halo-benzenesulfonic, toluenesulfonic, naphthalenesulfonic acids or sulfanilic acid, and also methionine, tryptophan, lysine or arginine, as well as ascorbic acid.

In view of the close relationship between the compounds (especially of formula I) in free form and in the form of their salts, including those salts that can be used as intermediates, for example in the purification or identification of the novel compounds, and of their solvates, any reference hereinbefore and hereinafter to the free compounds is to be understood as referring also to the corresponding salts, and the solvates thereof, for example hydrates, as appropriate and expedient.

The compounds of formula A, B, C, D, I, II, III, IV, V or VI especially those wherein R₅ is hydrogen, possess valuable pharmacological properties.

In the case of the groups of radicals or compounds mentioned hereinbefore and hereinafter, general definitions may, insofar as appropriate and expedient, be replaced by the more specific definitions stated hereinbefore and hereinafter.

Preference is given to a compounds of formula I, II, III, IV, V, VI wherein
R₁ and R₂ independently of each other are lower alkyl, lower alkyl substituted by halogen, C₆-C₁₄aryl, hydroxy, lower alkoxy, phenyl-lower alkoxy, phenyloxy, lower alkanoyloxy, benzoyloxy, amino, lower alkylamino, lower alkanoylamino, phenyl-lower alkylamino, N,N-di-lower alkylamino, N,N-di-(phenyl-lower alkyl)amino, cyano, mercapto, lower alkylthio, carboxy, lower alkoxycarbonyl, carbamoyl, N-lower alkylcarbamoyl, N,N-di-lower alkylcarbamoyl, sulfo, lower alkanesulfonyl, lower alkoxysulfonyl, aminosulfonyl, N-lower - alkylaminosulfonyl or N,N-di-lower alkylaminosulfonyl; halogen; lower alkoxy; C₆-C₁₄aryloxy; C₆-C₁₄aryl-lower alkoxy; lower alkanoyloxy; C₆-C₁₄arylcarbonyloxy; amino monosubstituted or disubstituted by lower alkyl, C₆-C₁₄aryl, C₆-C₁₄aryl-lower alkyl, lower alkanoyl or C₆-C₁₂-arylcarbonyl; cyano; nitro; mercapto; lower alkylthio; C₆-C₁₄arylthio; C₆-C₁₄aryl-lower alkylthio; lower alkanoylthio; C₆-C₁₄aryl-lower alkanoylthio; carboxy; lower alkoxycarbonyl, C₆-C₁₄aryl-lower alkoxycarbonyl; C₆-C₁₄aryloxycarbonyl; carbamoyl; carbamoyl N-mono- or N,N-disubstituted by lower alkyl, C₆-C₁₄aryl or C₆-C₁₄aryl-lower alkyl; sulfo; C₆-C₁₄arylsulfonyl; C₆-C₁₄aryl-lower alkanesulfonyl; lower alkanesulfonyl; or aminosulfonyl N-mono- or N,N-disubstituted by lower alkyl, C₆-C₁₄aryl or C₆-C₁₄aryl-lower alkyl, wherein C₆-C₁₄aryl is an aryl radical with 6 to 12 carbon atoms in the ring system, which may be unsubstituted or substituted by halogen, phenyl or naphthyl, hydroxy, lower alkoxy, phenyl-lower alkoxy, phenyloxy, lower alkanoyloxy, benzoyloxy, amino, lower alkylamino, lower alkanoylamino, phenyl-lower alkylamino, N,N-di-lower alkylamino, N,N-di-(phenyl-lower alkyl)amino, cyano, mercapto, lower alkylthio, carboxy, lower alkoxycarbonyl, carbamoyl, N-lower alkylcarbamoyl, N,N-di-lower alkylcarbamoyl, sulfo, lower alkanesulfonyl, lower alkoxysulfonyl, aminosulfonyl, N-lower alkylaminosulfonyl or N,N-di-lower alkylaminosulfonyl;
n and m are independently of each other 0 or 1 or 2, preferably 0;
R₃, R₄, R₈, R₁₀ are independently of each other hydrogen, lower alkyl, lower alkenyl or lower alkadienyl, which are each unsubstituted or monosubstituted or polysubsituted, preferably monosubstituted or disubstituted by a substituent independently selected from lower alkyl; hydroxy; lower alkoxy, which may be unsubstituted or mono-, di-, or trisubstituted by (i) heterocyclyl with 4 to 12 ring atoms, which may be unsaturated, wholly saturated, or partly saturated, is monocyclic or bicyclic and may contain up to three heteroatoms selected from nitrogen, oxygen and sulfur, and is most especially pyrrolyl, for example 2-pyrrolyl or 3-pyrrolyl, pyridyl, for example 2-, 3- or 4-pyridyl, or in a broader sense also thienyl, for example 2- or 3-thienyl, or furyl, for example 2-furyl, indolyl, typically 2- or 3-indolyl, quinolyl, typically 2- or 4-quinolyl, isoquinolyl, typically 3- or 5-isoquinolyl, benzofuranyl, typically 2-benzofuranyl, chromenyl, typically 3-chromenyl, benzothienyl, typically 2- or 3-benzothienyl; imidazolyl, typically 1- or 2-imidazolyl, pyrimidinyl, typically 2-or 4-pyrimidinyl, oxazolyl, typically 2-oxazolyl, isoxazolyl, typically 3-isoxazolyl, thiazolyl, typically 2-thiazolyl, benzimidazolyl, typically 2-benzimidazolyl, benzoxazolyl, typically 2-benzoxazolyl, quinazolyl, typically 2-quinazolinyl, 2-tetrahydrofuryl, 4-tetrahydrofuryl, 4-tetrahydropyranyl, 1-, 2- or 3-pyrrolidyl, 1-, 2-, 3-, or 4-piperidyl, 1-, 2-or 3-morpholinyl, 2- or 3-thiomorpholinyl, 2-piperazinyl or N,N'-bis-lower alkyl-2-piperazinyl, (ii) by halogen, (iii) by hydroxy or (iv) by lower alkoxy; phenoxy; phenyl-lower alkoxy; heterocyclyloxy, wherein heterocyclyl is pyrrolyl, for example 2-pyrrolyl or 3-pyrrolyl, pyridyl, for example 2-, 3- or 4-pyridyl, or in a broader sense also thienyl, for example 2- or 3-thienyl, or furyl, for example 2-furyl, indolyl, typically 2- or 3-indolyl, quinolyl, typically 2- or 4-quinolyl, isoquinolyl, typically 3- or 5-isoquinolyl, benzofuranyl, typically 2-benzofuranyl, chromenyl, typically 3-chromenyl, benzothienyl, typically 2- or 3-benzothienyl; imidazolyl, typically 1- or 2-imidazolyl, pyrimidinyl, typically 2-or 4-pyrimidinyl, oxazolyl, typically 2-oxazolyl, isoxazolyl, typically 3-isoxazolyl, thiazolyl, typically 2-thiazolyl, benzimidazolyl, typically 2-benzimidazolyl, benzoxazolyl, typically 2-benzoxazolyl, quinazolyl, typically 2-quinazolinyl, 2-tetrahydrofuryl, 4-tetrahydrofuryl, 2- or 4-tetrahydropyranyl, 1-, 2- or 3-pyrrolidyl, 1-, 2-, 3-, or 4-piperidyl, 1-, 2-or 3-morpholinyl, 2- or 3-thiomorpholinyl, 2-piperazinyl or N,N'-bis-lower alkyl-2-piperazinyl, such as especially 2- or 4-tetrahydropyranyloxy; lower alkanoyloxy; carboxy; lower alkoxycarbonyl; phenyl-lower alkoxycarbonyl; mercapto; lower alkylthio; phenylthio; halogen; halogen-lower alkyl; oxo (except in the 1-position, because otherwise acyl); azido; nitro; cyano; amino; mono-lower alkylamino; di-lower alkylamino; pyrrolidino; imidazol-1-yl; piperidino; piperazino; 4-lower alkylpiperazino; morpholino; thiomorpholino; diphenylamino or dibenzylamino unsubstituted or substituted in the phenyl part by lower alkyl, lower alkoxy, halogen and/or nitro; lower alkoxycarbonylamino; phenyl-lower alkoxycarbonylamino unsubstituted or substituted in the phenyl part by lower alkyl or lower alkoxy; fluorenylmethoxycarbonylamino; amino-lower alkyl; monosubstituted or disubstituted amino-lower alkyl, wherein the amino substituent is selected from lower alkyl, hydroxy-lower alkyl, C₃-C₈cycloalkyl, amino-lower alkyl, N-mono- or N,N-di(-lower alkyl)amino-lower alkyl, amino, N-mono- or N,N-di-lower alkylamino and N-mono- or N,N-di-(hydroxy-lower alkyl)amino; pyrrolidino-lower alkyl; piperidino-lower alkyl; piperazino-lower alkyl; 4-lower alkylpiperazino-lower alkyl; imidazol-1-yl-lower alkyl; morpholino-lower alkyl; thiomorpholino-lower alkyl; S-oxo-thiomorpholino-lower alkyl; S,S-dioxothiomorpholino-lower alkyl; lower alkylendioxy; sulfamoyl; sulfo; carbamoyl; ureido; guanidino; cyano; aminocarbonyl (carbamoyl) and aminocarbonyloxy, which are substituted by one or two radicals on the nitrogen, wherein the amino substituents are selected independently of one another from the group comprising lower alkyl, hydroxy-lower alkyl, C₃-C₈cycloalkyl, amino-lower alkyl, N-mono- or N,N-di(-lower alkyl)amino-lower alkyl, amino, N-mono- or N,N-di-lower alkylamino and N-mono- or N,N-di-(hydroxy-lower alkyl)amino; pyrrolidinocarbonyl; piperidinocarbonyl; piperazino-carbonyl; 4-lower alkylpiperazinocarbonyl; imidazolinocarbonyl; morpholinocarbonyl; thiomorpholinocarbonyl; S-oxo-thiomorpholinocarbonyl; and S,S-dioxothiomorpholino;
phenyl, naphthyl, phenyl-lower alkyl or phenyl-lower alkenyl with a terminal phenyl radical, which is unsubstituted or monosubstituted or disubstituted by the radicals named above as substituents of lower alkyl, lower alkenyl or lower alkadienyl;
or heterocyclyl-lower alkyl, wherein heterocyclyl is pyrrolyl, for example 2-pyrrolyl or 3-pyrrolyl, pyridyl, for example 2-, 3- or 4-pyridyl, or in a broader sense also thienyl, for example 2- or 3-thienyl, or furyl, for example 2-furyl, indolyl, typically 2- or 3-indolyl, quinolyl, typically 2- or 4-quinolyl, isoquinolyl, typically 3- or 5-isoquinolyl, benzofuranyl, typically 2-benzofuranyl, chromenyl, typically 3-chromenyl, benzothienyl, typically 2- or 3-benzothienyl; imidazolyl, typically 1- or 2-imidazolyl, pyrimidinyl, typically 2-or 4-pyrimidinyl, oxazolyl, typically 2-oxazolyl, isoxazolyl, typically 3-isoxazolyl, thiazolyl, typically 2-thiazolyl, benzimidazolyl, typically 2-benzimidazolyl, benzoxazolyl, typically 2-benzoxazolyl, quinazolyl, typically 2-quinazolinyl, 2-tetrahydrofuryl, 4-tetrahydrofuryl, 2- or 4-tetrahydropyranyl, 1-, 2- or 3-pyrrolidyl, 1-, 2-, 3-, or 4-piperidyl, 1-, 2-or 3-morpholinyl, 2- or 3-thiomorpholinyl, 2-piperazinyl or N,N'-bis-lower alkyl-2-piperazinyl, which in each case are unsubstituted or monosubstituted or disubstituted by the radicals named above as substituents of lower alkyl, lower alkenyl, or lower alkadienyl;
or acyl of the subformula Y-C(=W)-, wherein W is oxygen and Y is hydrogen, R°, R°-O-, R°HN-, or R°R°N- (wherein the radicals R° may be the same or different),
or
acyl of the subformula R°-SO₂-,
whereby R₄ may also be absent for the compound of formula II;
or
R₄ is absent for compounds of formula II, hydrogen or CH₃ for compounds of formula I, and R₃ is acyl of the subformula Y-C(=W)-, wherein W is oxygen and Y is hydrogen, R°, R°-O-, R°HN-, or R°R°N- (wherein the radicals R° may be the same or different),
or
is acyl of the subformula R°-SO₂-,
wherein R⁰ in the said radicals has the following meanings: substituted or unsubstituted lower alkyl, especially methyl or ethyl, amino-lower alkyl hydroxy-lower alkyl, wherein the amino group is unprotected or is protected by a conventional amino protecting group - especially by lower alkoxycarbonyl, typically tert-lower alkoxycarbonyl, for example tert-butoxycarbonyl - e.g. aminomethyl, R,S-, R- or preferably S-1-aminoethyl, tert-butoxycarbonylaminomethyl or R,S-, R-, or preferably S-1-(tert-butoxycarbonylamino)ethyl, carboxy-lower alkyl, typically 2-carboxyethyl, lower alkoxycarbonyl-lower alkyl, typically 2-(tert-butoxycarbonyl)ethyl, cyano-lower alkyl, typically 2-cyanoethyl, tetrahydropyranyloxy-lower alkyl, typically 4-(tetrahydropyranyl)oxymethyl, morpholino-lower alkyl, typically 2-(morpholino)ethyl, phenyl, lower alkylphenyl, typically 4-methylphenyl, lower alkoxyphenyl, typically 4-methoxyphenyl, imidazolyl-lower alkoxyphenyl, typically 4-[2-(imidazol-1-yl)ethyl)oxyphenyl, carboxyphenyl, typically 4-carboxyphenyl, lower alkoxycarbonylphenyl, typically 4-ethoxycarbonylphenyl or 4-methoxyphenyl, halogen-lower alkylphenyl, typically 4-chloromethylphenyl, pyrrolidinophenyl, typically 4-pyrrolidinophenyl, imidazol-1-ylphenyl, typically 4-(imidazolyl-1-yl)phenyl, piperazinophenyl, typically 4-piperazinophenyl, (4-lower alkylpiperazino)phenyl, typically 4-(4-methylpiperazino)phenyl, morpholinophenyl, typically 4-morpholinophenyl, pyrrolidino-lower alkylphenyl, typically 4-pyrrolidinomethylphenyl, imidazol-1-yl-lower alkylphenyl, typically 4-(imidazolyl-1-ylmethyl)phenyl, piperazino-lower alkylphenyl, typically 4-piperazinomethylphenyl, (4-lower alkylpiperazinomethyl)-phenyl, typically 4-(4-methylpiperazinomethyl)phenyl, morpholino-lower alkylphenyl, typically 4-morpholinomethylphenyl, piperazinocarbonylphenyl, typically 4-piperazinocarbonylphenyl, or (4-lower alkylpiperazino)phenyl, typically 4-(4-methylpiperazino)phenyl.
p is 0 if R₄ is absent, or is 1 if R₃ and R₄ are both present and in each case are one of the aforementioned radicals (for compounds of formula II);
R₅ is hydrogen or lower alkyl, especially hydrogen,
X stands for 2 hydrogen atoms, for O, or for 1 hydrogen atom and hydroxy; or for 1 hydrogen atom and lower alkoxy;
Z is hydrogen or especially lower alkyl, most especially methyl;
and for compounds for formula II, either the two bonds characterised by wavy lines are preferably absent in ring A and replaced by 4 hydrogen atoms, and the two wavy lines in ring B each, together with the respective parallel bond, signify a double bond;
or also the two bonds characterised by wavy lines are absent in ring B and replaced by a total of 4 hydrogen atoms, and the two wavy lines in ring A each, together with the respective parallel bond, signify a double bond;
or both in ring A and in ring B all of the 4 wavy bonds are absent and are replaced by a total of 8 hydrogen atoms;
or a salt thereof, if at least one salt-forming group is present.
Particular preference is given to a compound of formula I wherein;
m and n are each 0;
R₃ and R₄ are independently of each other
hydrogen,
lower alkyl unsubstituted or mono- or disubstituted, especially monosubstituted, by radicals selected independently of one another from carboxy; lower alkoxycarbonyl; and cyano;;
or
R₄ is hydrogen or -CH₃, and
R₃ is as defined above or preferably R₃ is,
acyl of the subformula R°-CO, wherein R° is lower alkyl; amino-lower alkyl, wherein the amino group is present in unprotected form or is protected by lower alkoxycarbonyl; tetrahydropyranyloxy-lower alkyl; phenyl; imidazolyl-lower alkoxyphenyl; carboxyphenyl; lower alkoxycarbonylphenyl; halogen-lower alkylphenyl; imidazol-1-ylphenyl; pyrrolidino-lower alkylphenyl; piperazino-lower alkylphenyl; (4-lower alkylpiperazinomethyl)phenyl; morpholino-lower alkylphenyl; piperazinocarbonylphenyl; or (4-lower alkylpiperazino)phenyl;
or is acyl of the subformula R°-O-CO-, wherein R° is lower alkyl;
or is acyl of the subformula R°HN-C(=W)-, wherein W is oxygen and R° has the following meanings: morpholino-lower alkyl, phenyl, lower alkoxyphenyl, carboxyphenyl, or lower alkoxycarbonylphenyl;
or R₃ is lower alkylphenylsulfonyl, typically 4-toluenesulfonyl;
further specific examples of preferred R₃ groups are described below for the preferred compounds of formula II,
R₅ is hydrogen or lower alkyl, especially hydrogen,
X stands for 2 hydrogen atoms or for O;
Z is methyl or hydrogen;
or a salt thereof, if at least one salt-forming group is present.
Particular preference is given to a compound of formula II wherein
m and n are each 0;
R₃ and R₄ are independently of each other
hydrogen,
lower alkyl unsubstituted or mono- or disubstituted, especially monosubstituted, by radicals selected independently of one another from carboxy; lower alkoxycarbonyl; and cyano; whereby R₄ may also be absent;
or
R₄ is absent, and
R₃ is acyl from the subformula R°-CO, wherein R° is lower alkyl, especially methyl or ethyl; amino-lower alkyl, wherein the amino group is unprotected or protected by lower alkoxycarbonyl, typically tert-lower alkoxycarbonyl, for example tert-butoxycarbonyl, e.g. aminomethyl, R,S-, R-, or preferably S-1-aminoethyl, tert-butoxycarbonylaminomethyl or R,S-, R-, or preferably S-1-(tert-butoxycarbonylamino)ethyl; tetrahydropyranyloxy-lower alkyl, typically 4-(tetrahydropyranyl)oxymethyl; phenyl; imidazolyl-lower alkoxyphenyl, typically 4-[2-(imidazol-1-yl)ethyl)oyxphenyl; carboxyphenyl, typically 4-carboxyphenyl; lower alkoxycarbonylphenyl, typically 4-methoxy- or 4-ethoxycarbonylphenyl; halogen-lower alkylphenyl, typically 4-chloromethylphenyl; imidazol-1-ylphenyl, typically 4-(imidazolyl-1-yl)-phenyl; pyrrolidino-lower alkylphenyl, typically 4-pyrrolidinomethylphenyl; piperazino-lower alkylphenyl, typically 4-piperazinomethylphenyl; (4-lower alkylpiperazinomethyl)phenyl, typically 4-(4-methylpiperazinomethyl)phenyl; morpholino-lower alkylphenyl, typically 4-morpholinomethylphenyl; piperazinocarbonylphenyl, typically 4-piperazinocarbonylphenyl; or (4-lower alkylpiperazino)phenyl, typically 4-(4-methylpiperazino)phenyl;
or is acyl of the subformula R°-O-CO-, wherein R° is lower alkyl;
or is acyl of the subformula R°HN-C(=W)-, wherein W is oxygen and R° has the following preferred meanings: morpholino-lower alkyl, typically 2-morpholinoethyl, phenyl, lower alkoxyphenyl, typically 4-methoxyphenyl or 4-ethoxyphenyl, carboxyphenyl, typically 4-carboxyphenyl, or lower alkoxycarbonylphenyl, typically 4-ethoxycarbonylphenyl;
or is lower alkylphenylsulfonyl, typically 4-toluenesulfonyl;
p is 0 if R₄ is absent, or is 1 if R₃ and R₄ are both present and in each case are one of the aforementioned radicals;
R₅ is hydrogen or lower alkyl, especially hydrogen,
X stands for 2 hydrogen atoms or for O;
Z is methyl or hydrogen;
and either the two bonds characterised by wavy lines are preferably absent in ring A and replaced by 4 hydrogen atoms, and the two wavy lines in ring B each, together with the respective parallel bond, signify a double bond;
or also the two bonds characterised by wavy lines are absent in ring B and replaced by a total of 4 hydrogen atoms, and the two wavy lines in ring A each, together with the respective parallel bond, signify a double bond;
or both in ring A and in ring B all of the 4 wavy bonds are absent and are replaced by a total of 8 hydrogen atoms;
or a salt thereof, if at least one salt-forming group is present.

Most especially preferred compounds of formula II are selected from; 8,9,10,11-Tetrahydrostaurosporine;
N-[4-(4-methylpiperaziN-1-ylmethyl)benzoyl]-1,2,3,4-tetrahydrostaurosporine;
N-(4-chloromethylbenzoyl)-1,2,3,4-tetrahydrostaurosporine;
N-(4-(pyrrolidin-1-ylmethyl)benzoyl)-1,2,3,4-tetrahydrostaurosporine;
N-(4-(morpholin-4-ylmethyl)benzoyl)-1,2,3,4-tetrahydrostaurosporine;
N-(4-(piperazin-1-ylmethyl)benzoyl)-1,2,3,4-tetrahydrostaurosporine;
N-ethyl-1,2,3,4-tetrahydrostaurosporine;
N-tosyl-1,2,3,4-tetrahydrostaurosporine;
N-triflouroacetyl-1,2,3,4-tetrahydrostaurosporine;
N-[4-(2-imidazol-1-yl-ethoxy)benzoyl]-1,2,3,4-tetrahydrostaurosporine;
N-methoxycarbonylmethyl-1,2,3,4-tetrahydrostaurosporine;
N-carboxymethyl-1,2,3,4-tetrahydrostaurosporine;
N-terephthaloylmethyl ester-1,2,3,4-tetrahydrostaurosporine;
N-terephthaloyl-1,2,3,4-tetrahydrostaurosporine;
N-(4-ethylpiperazinylcarbonylbenzoyl)-1,2,3,4-tetrahydrostaurosporine;
N-(2-cyanoethyl)-1,2,3,4-tetrahydrostaurosporine;
N-benzoyl-1,2,3,4-tetrahydrostaurosporine;
N,N-dimethyl -1,2,3,4-tetrahydrostaurosporinium iodide;
N-BOC-glycyl-1,2,3,4-tetrahydrostaurosporine;
N-glycyl-1,2,3,4-tetrahydrostaurosporine;
N-(3-(tert-butoxycarbonyl)propyl)-1,2,3,4-tetrahydrostaurosporine;
N-(3-carboxypropyl)-1,2,3,4-tetrahydrostaurosporine;
N-(4-imidazol-1-yl)benzoyl]-1,2,3,4-tetrahydrostaurosporine;
N-[(tetrahydro-2h-pyran-4-yloxy)acetyl]-1,2,3,4-tetrahydrostaurosporine;
N-BOC-I-alanyl-1,2,3,4-tetrahydrostaurosporine;
N-I-alanyl-1,2,3,4-tetrahydrostaurosporine hydrochloride;
N-methyl-1,2,3,4-tetrahydro-6-methylstaurosporine;
N-(4-carboxyphenylaminocarbonyl)-1,2,3,4-tetrahydrostaurosporine;
N-(4-ethylphenylaminocarbonyl)-1,2,3,4-tetrahydrostaurosporine;
N-(N-phenylaminocarbonyl)-1,2,3,4-tetrahydrostaurosporine;
N-(N-[2-(1-morpholino)ethyl]aminocarbonyl)-1,2,3,4-tetrahydrostaurosporine;
N-(N-[4-methoxyphenyl]aminocarbonyl)-1,2,3,4-tetrahydrostaurosporine;
1,2,3,4-tetrahydro-6-methylstaurosporine;
N-BOC-1,2,3,4-tetrahydrostaurosporine;
N-BOC-1,2,3,4-tetrahydro-6-methylstaurosporine;
N-BOC-1,2,3,4-tetrahydro-6-methyl-7-oxo-staurosporine;
1,2,3,4,8,9,10,11-octahydrostaurosporine;
or a pharmaceutically acceptable salt thereof, if at least one salt-forming group is present.

Most especially preferred is the compound of formula I designated 1,2,3,4-tetrahydrostaurosporine, or a (particularly pharmaceutically acceptable) salt thereof (here, m und n in formula I are 0, R₃ is hydrogen, R₄ is absent, provided no salt is present (p = 0), or is hydrogen if a salt is present (p = 1), R₅ is hydrogen, the two bonds represented by wavy lines are absent in Ring A and are replaced by a total of 4 hydrogen atoms and the two bonds represented by wavy lines in Ring B are in each case a double bond together with the parallel bonds, X stands for 2 hydrogen atoms, and Z is methyl).

Most especially preferred are the compounds of formula A wherein;
A) X= O; R₁, R₂, R₅ = H; Q= -(CH₂)₂-O-CH(CH₂)OH-(CH₂)₂-
B) X= O; R₁, R₂, R₅ = H; Q= -(CH₂)₂-O-CH(CH₂N(CH₃)₂)-(CH₂)₂-
C) X= 2 hydrogen atoms; R₁, R₂, R₅ = H;

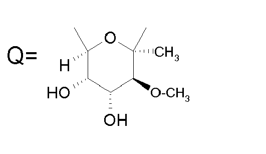

Most especially preferred are the compounds of formula I wherein;
A) X= 2 hydrogen atoms; R₁,R₂, R₃, R₅ = H; R₄= CH₃; Z=CH₃ (staurosporine)
B) X= 1 hydrogen and 1 hydroxy atoms in (R) or (S) isomeric form; R₁,R₂, R₃,R₅ = H; R₄= CH₃; Z=CH₃ (UCN-01 and UCN-02)
C) X= 2 hydrogen atoms; R₁,R₂, R₅ = H; R₄= CH₃; R₃,= benzoyl; Z=CH₃ (CGP41251 or PKC412 or MIDOSTAURIN)
D) X= O; R₁,R₂, R₅ = H; R₃,= CH₃; R₄= ethyloxycarbonyl; Z=CH₃ (NA 382 ; CAS=143086-33-3)
E) X= 1 hydrogen and 1 hydroxy atom; R₁, R₂, R₅ = H; R₃= CH₃; Z=CH₃; and R₄ is selected from -(CH₂)₂OH; -CH₂CH(OH)CH₂OH; -CO(CH₂)₂CO₂Na; -(CH₂)₃CO₂H; - COCH₂N(CH₃)₂; 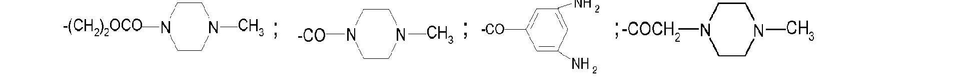
F) X= 2 hydrogen atoms; R₁, R₂, R₅ = H; R₃= CH₃; Z=CH₃; and R₄ is selected from N-[0-(tetrahydropyran-4-yl )-D-lactoyl]; N-[2-methyl-2-(tetrahydropyran-4-yloxy)-propionyl; N-[0-(tetrahydropyran-4-yl )-L-lactoyl]; N-[0-(tetrahydropyran-4-yl)-D-lactoyl]; N-[2-(tetrahydro-pyran-4-yloxy)-acetyl)]
G) X=O; R₁, R₂, R₅ = H; R₃= CH₃; Z=CH₃; and R₄ is selected from N-[0-(tetrahydropyran-4-yl )-D-lactoyl]; N-[2-(tetrahydro-pyran-4-yloxy)-acetyl)]
H) X=1 hydrogen and 1 hydroxy atom ; R₁, R₂, R₅ = H; R₃= CH₃; Z=CH₃; and R₄ is selected from N-[0-(tetrahydropyran-4-yl)-D-lactoyl]; N-[2-(tetrahydro-pyran-4-yloxy)-acetyl)]

The abbreviation "CAS" means the CHEMICAL ABSTRACTS registry number.

The most preferred compounds of formula I e.g. MIDOSTAURIN [International Nonproprietary Name] are covered and have been specifically described by the European patent No. 0 296 110 published on December 21, 1988, as well as in US patent No. 5;093,330 published on March 3, 1992, and Japanese Patent No. 2 708 047. Other preferred compounds are covered and described by the patent applications WO 95/32974 and WO 95/32976 both published on December 7, 1995. All the compounds described in these documents are incorporated into the present application by reference.

Most especially preferred are the compounds of formula III wherein;
A) X= 2 hydrogen atoms; R₁,R₂, R₅ = H; R₆= CH₃; R₇= methyloxycarbonyl; Z=H (2-methyl K252a)
B) X= 2 hydrogen atoms; R₁,R₂, R₅, R₆ = H; R₇= methyloxycarbonyl; Z= H (K-252a)
C) X= 2 hydrogen atoms; R₁,R₂, R₅, R₆ = H; R₇= methyloxycarbonyl; Z= CH₃ (KT-5720)

Most especially preferred are the compounds of formula IV wherein;
A) X= O; R₁, R₂, R₅ = H; R₉= CH₂-NMe₂; R₈= CH₃ ; m'=n'=2
B) X= O; R₁, R₂, R₅ = H; R₉= CH₂-NH₂; R₈= CH₃ ; m'=2; n'=1 (Ro-31-8425;
   CAS=151342-35-7)

Most especially preferred are the compounds of formula V wherein;
A) X= O; R₁, R₂, R₅ = H; R₈= CH₃; R₁₀= -(CH₂)₃-NH₂; (Ro-31-7549; CAS=138516-31)
B) X= O; R₁, R₂, R₅ = H; R₈= CH₃; R₁₀= -(CH₂)₃-S-(C=NH)-NH₂; (Ro-31-8220 ;
   CAS=125314-64-9))
C) X= O; R₁, R₂, R₅ = H; R₈= CH₃; R₁₀= -CH₃;

Most especially preferred are the compounds of formula VI wherein;
A) X= 2 hydrogen atoms; R₁,R₂, R₅ = H; R₄= CH₃; Z=CH₃ ; R₃ selected from methyl or (C₁-C₁₀)alkyl, arylmethyl, C₆H₂CH₂-

STAUROSPORINE DERIVATIVES and their manufacturing process have been specifically described in many prior documents, well known by the man skilled in the art.

Compounds of formula A, B, C, D and their manufacturing process have for instance, been described in the European patents No. 0 657 458 published on June 14, 1995, in the European patents No. 0 624 586 published on November 17, 1994, in the European patents No. 0 470 490 published on February 12, 1992, in the European patents No. 0 328 026 published on August 16, 1989, in the European patents No. 0 384 349 published on August 29, 1990, as well as in many publications such as Barry M. Trost* and Wiping Tang Org. Lett., 3(21), 3409-3411.

Compounds of formula I and their manufacturing processes have specifically been described in the European patents No. 0 296 110 published on December 21, 1988, as well as in US patent No. 5;093,330 published on March 3, 1992, and Japanese Patent No. 2 708 047. Compounds of formula I having a tetrahydropyran-4-yl)-lactoyl substitution on R₄ have been described in the European patent No. 0 624 590 published on November 17, 1994. Other compounds have been described in the European patent No. 0 575 955 published December 29, 1993, European patent No. 0 238 011 published on September 23, 1987 (UCN-O1), International patent application EP98/04141 published as WO99/02532 on July 03, 1998.

Compounds of formula II and their manufacturing processes have specifically been described in the European patents No. 0 296 110 published on December 21, 1988, as well as in US patent No. 5;093,330 published on March 3, 1992, and Japanese Patent No. 2 708 047.

Compounds of formula III and their manufacturing processes have specifically been described in the patent applications claiming the priority of the US patent application US 920102 filed on July 24, 1992. (i.e European patents No. 0 768 312 published on April 16, 1997, No. 1 002 534 published May 24, 2000, No. 0 651 754 published on May 10, 1995).

Compounds of formula IV and their manufacturing processes have specifically been described in the patent applications claiming the priority of the British patent applications GB 9309602 and GB 9403249 respectively filed on May 10, 1993, and on February 21, 1994. (i.e European patents No. 0 624 586 published on November 17, 1994, No. 1 002 534 published May 24, 2000, No. 0 651 754 published on May 10, 1995).

Compounds of formula V and their manufacturing processes have specifically been described in the patent applications claiming the priority of the British patent applications GB 8803048, GB 8827565, GB 8904161 and GB 8928210 respectively filed on February 10, 1988, November 25, 1988, February 23, 1989 and December 13, 1989. (i.e European patents No. 0 328 026 published on August 16, 1989, and No. 0 384 349 published August 29, 1990).

Compounds of formula VI and their manufacturing processes have specifically been described in the patent applications claiming the priority of the US patent applications 07/777,395 (Con), filed on October 10, 1991 (i.e International patent application WO 93/07153 published on April 15, 1993).

In each case where citations of patent applications or scientific publications are given in particular for the STAUROSPORINE DERIVATIVE compounds, the subject-matter of the final products, the pharmaceutical preparations and the claims are hereby incorporated into the present application by reference to these publications.

The structure of the active agents identified by code nos., generic or trade names may be taken from the actual edition of the standard compendium "The Merck Index" or from databases, e.g. Patents International (e.g. IMS World Publications). The corresponding content thereof is hereby incorporated by reference.

The preferred STAUROSPORINE DERIVATIVE according to the invention is *N-*[(9*S*,10*R*,11*R*,13*R*)-2,3,10,11,12,13-hexahydro-10-methoxy-9-methyl-1-oxo-9,13-epoxy-1*H*,9*H*-diindolo[1,2,3-gh:3',2',1'-lm]pyrrolo[3,4-j][1,7]benzodiazonin-11-yl]-*N-*methylbenzamide of the formula (VII): 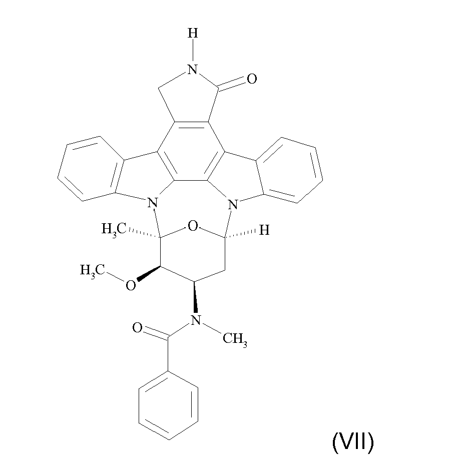 or a salt thereof, (hereinafter: "Compound of formula VII or MIDOSTAURIN").

Compound of formula VII is also known as MIDOSTAURIN [International Nonproprietary Name] or PKC412.

MIDOSTAURIN is a derivative of the naturally occurring alkaloid staurosporine, and has been specifically described in the European patent No. 0 296 110 published on December 21, 1988, as well as in US patent No. 5;093,330 published on March 3, 1992, and Japanese Patent No. 2 708 047.

HDAI compounds of particular interest for use in the inventive combination are hydroxamate compounds described by the formula X: 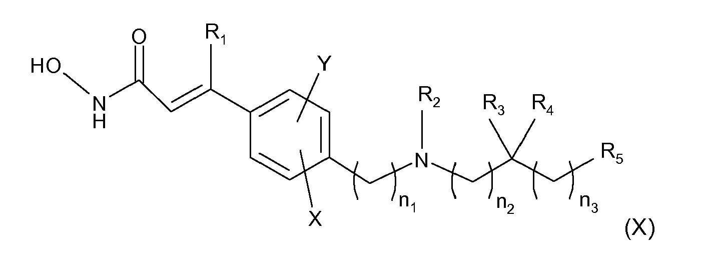 wherein
R₁ is H, halo, or a straight chain C₁-C₆ alkyl (especially methyl, ethyl or n-propyl, which methyl, ethyl and n-propyl substituents are unsubstituted or substituted by one or more substituents described below for alkyl substituents);
R₂ is selected from H, C₁-C₁₀ alkyl, (preferably C₁-C₆ alkyl, e.g. methyl, ethyl or - CH₂CH₂-OH), C₄ - C₉ cycloalkyl, C₄ - C₉ heterocycloalkyl, C₄ - C₉ heterocycloalkylalkyl, cycloalkylalkyl (e.g., cyclopropylmethyl), aryl, heteroaryl, arylalkyl (e.g. benzyl), heteroarylalkyl (e.g. pyridylmethyl), -(CH₂)ₙC(O)R₆, - (CH₂)ₙOC(O)R₆, amino acyl, HON-C(O)-CH=C(R₁)-aryl-alkyl- and -(CH₂)ₙR₇;
R₃ and R₄ are the same or different and independently H, C₁-C₆ alkyl, acyl or acylamino, or R₃ and R₄ together with the carbon to which they are bound represent C=O, C=S, or C=NR₈, or R₂ together with the nitrogen to which it is bound and R₃ together with the carbon to which it is bound can form a C₄ - C₉ heterocycloalkyl, a heteroaryl, a polyheteroaryl, a non-aromatic polyheterocycle, or a mixed aryl and non-aryl polyheterocycle ring;
R₅ is selected from H, C₁-C₆ alkyl, C₄ - C₉ cycloalkyl, C₄ - C₉ heterocycloalkyl, acyl, aryl, heteroaryl, arylalkyl (e.g. benzyl), heteroarylalkyl (e.g. pyridylmethyl), aromatic polycycles, non-aromatic polycycles, mixed aryl and non-aryl polycycles, polyheteroaryl, non-aromatic polyheterocycles, and mixed aryl and non-aryl polyheterocycles;
n, n₁, n₂ and n₃ are the same or different and independently selected from 0 - 6, when n₁ is 1-6, each carbon atom can be optionally and independently substituted with R₃ and/or R₄;
X and Y are the same or different and independently selected from H, halo, C₁-C₄ alkyl, such as CH₃ and CF₃, NO₂, C(O)R₁, OR₉, SR₉, CN, and NR₁₀R₁₁;
R₆ is selected from H, C₁-C₆ alkyl, C₄ - C₉ cycloalkyl, C₄ - C₉ heterocycloalkyl, cycloalkylalkyl (e.g., cyclopropylmethyl), aryl, heteroaryl, arylalkyl (e.g., benzyl, 2- phenylethenyl), heteroarylalkyl (e.g., pyridylmethyl), OR₁₂, and NR₁₃R₁₄;
R₇ is selected from OR₁₅, SR₁₅, S(O)R₁₆, SO₂R₁₇, NR₁₃R₁₄, and NR₁₂SO₂R₆;
R₈ is selected from H, OR₁₅, NR₁₃R₁₄, C₁-C₆ alkyl, C₄ - C₉ cycloalkyl, C₄ - C₉ heterocycloalkyl, aryl, heteroaryl, arylalkyl (e.g., benzyl), and heteroarylalkyl (e.g., pyridylmethyl);
Rg is selected from C₁ - C₄ alkyl, for example, CH₃ and CF₃, C(O)-alkyl, for example C(O)CH₃, and C(O)CF₃;
R₁₀ and R₁₁ are the same or different and independently selected from H, C₁-C₄ alkyl, and -C(O)-alkyl;
R₁₂ is selected from H, C₁-C₆ alkyl, C₄ - C₉ cycloalkyl, C₄ - C₉ heterocycloalkyl, C₄ - C₉ heterocycloalkylalkyl, aryl, mixed aryl and non-aryl polycycle, heteroaryl, arylalkyl (e.g., benzyl), and heteroarylalkyl (e.g., pyridylmethyl);
R₁₃ and R₁₄ are the same or different and independently selected from H, C₁-C₆ alkyl, C₄ - C₉ cycloalkyl, C₄ - C₉ heterocycloalkyl, aryl, heteroaryl, arylalkyl (e.g., benzyl), heteroarylalkyl (e.g., pyridylmethyl), amino acyl, or R₁₃ and R₁₄ together with the nitrogen to which they are bound are C₄ - C₉ heterocycloalkyl, heteroaryl, polyheteroaryl, non-aromatic polyheterocycle or mixed aryl and non-aryl polyheterocycle;
R₁₅ is selected from H, C₁-C₆ alkyl, C₄ - C₉ cycloalkyl, C₄ - C₉ heterocycloalkyl, aryl, heteroaryl, arylalkyl, heteroarylalkyl and (CH₂)ₘZR₁₂;
R₁₆ is selected from C₁-C₆ alkyl, C₄ - C₉ cycloalkyl, C₄ - C₉ heterocycloalkyl, aryl, heteroaryl, polyheteroaryl, arylalkyl, heteroarylalkyl and (CH₂)ₘZR₁₂;
R₁₇ is selected from C₁-C₆ alkyl, C₄ - C₉ cycloalkyl, C₄ - C₉ heterocycloalkyl, aryl, aromatic polycycles, heteroaryl, arylalkyl, heteroarylalkyl, polyheteroaryl and N R₁₃R₁₄; or a
m is an integer selected from 0 to 6; and Z is selected from O, NR₁₃, S and S(O), or a pharmaceutically acceptable salt thereof.

As appropriate, unsubstituted here means that there is no substituent or that the only substituents are hydrogen and the general terms and definitions used hereinbefore and hereinafter preferably have the following meanings for the histone deactylase inhibitors (HDAI):
Halogen is preferably fluorine, chlorine, bromine, or iodine, especially fluorine or chlorine.

Alkyl substituents include straight and branched C₁-C₆alkyl, unless otherwise noted. Examples of suitable straight and branched C₁-C₆alkyl substituents include methyl, ethyl, n-propyl, 2-propyl, n-butyl, sec-butyl, t-butyl, and the like. Unless otherwise noted, the alkyl substituents include both unsubstituted alkyl groups and alkyl groups that are substituted by one or more suitable substituents, including unsaturation (i.e. there are one or more double or triple C-C bonds), acyl, cycloalkyl, halo, oxyalkyl, alkylamino, aminoalkyl, acylamino and OR₁₅, for example, alkoxy. Preferred substituents for alkyl groups include halo, hydroxy, alkoxy, oxyalkyl, alkylamino, and aminoalkyl.

Cycloalkyl substituents include C₃-C₉ cycloalkyl groups, such as cyclopropyl, cyclobutyl, cyclopentyl, cyclohexyl and the like, unless otherwise specified. Unless otherwise noted, cycloalkyl substituents include both unsubstituted cycloalkyl groups and cycloalkyl groups that are substituted by one or more suitable substituents, including C₁-C₆ alkyl, halo, hydroxy, aminoalkyl, oxyalkyl, alkylamino, and OR₁₅, such as alkoxy. Preferred substituents for cycloalkyl groups include halo, hydroxy, alkoxy, oxyalkyl, alkylamino and aminoalkyl.

The above discussion of alkyl and cycloalkyl substituents also applies to the alkyl portions of other substituents, such as without limitation, alkoxy, alkyl amines, alkyl ketones, arylalkyl, heteroarylalkyl, alkylsulfonyl and alkyl ester substituents and the like.

Heterocycloalkyl substituents include 3 to 9 membered aliphatic rings, such as 4 to 7 membered aliphatic rings, containing from one to three heteroatoms selected from nitrogen, sulfur, oxygen. Examples of suitable heterocycloalkyl substituents include pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane. Unless otherwise noted, the rings are unsubstituted or substituted on the carbon atoms by one or more suitable substituents, including C₁-C₆ alkyl, C₄ - C₉ cycloalkyl, aryl, heteroaryl, arylalkyl (e.g., benzyl), and heteroarylalkyl (e.g., pyridylmethyl), halo, amino, alkyl amino and OR₁₅, for example alkoxy. Unless otherwise noted, nitrogen heteroatoms are unsubstituted or substituted by H, C₁-C₄ alkyl, arylalkyl (e.g., benzyl), and heteroarylalkyl (e.g., pyridylmethyl), acyl, aminoacyl, alkylsulfonyl, and arylsulfonyl.

Cycloalkylalkyl substituents include compounds of the formula -(CH₂)ₙ₅-cycloalkyl wherein n5 is a number from 1-6. Suitable alkylcycloalkyl substituents include cyclopentylmethyl-, cyclopentylethyl, cyclohexylmethyl and the like. Such substituents are unsubstituted or substituted in the alkyl portion or in the cycloalkyl portion by a suitable substituent, including those listed above for alkyl and cycloalkyl.

Aryl substituents include unsubstituted phenyl and phenyl substituted by one or more suitable substituents, including C₁-C₆ alkyl, cycloalkylalkyl (e.g., cyclopropylmethyl), O(CO)alkyl, oxyalkyl, halo, nitro, amino, alkylamino, aminoalkyl, alkyl ketones, nitrile, carboxyalkyl, alkylsulfonyl, aminosulfonyl, arylsulfonyl, and OR₁₅, such as alkoxy. Preferred substituents include including C₁-C₆ alkyl, cycloalkyl (e.g., cyclopropylmethyl), alkoxy, oxyalkyl, halo, nitro, amino, alkylamino, aminoalkyl, alkyl ketones, nitrile, carboxyalkyl, alkylsulfonyl, arylsulfonyl, and aminosulfonyl. Examples of suitable aryl groups include C₁-C₄alkylphenyl, C₁-C₄alkoxyphenyl, trifluoromethylphenyl, methoxyphenyl, hydroxyethylphenyl, dimethylaminophenyl, aminopropylphenyl, carbethoxyphenyl, methanesulfonylphenyl and tolylsulfonylphenyl.

Aromatic polycycles include naphthyl, and naphthyl substituted by one or more suitable substituents, including C₁-C₆ alkyl, alkylcycloalkyl (e.g., cyclopropylmethyl), oxyalkyl, halo, nitro, amino, alkylamino, aminoalkyl, alkyl ketones, nitrile, carboxyalkyl, alkylsulfonyl, arylsulfonyl, aminosulfonyl and OR₁₅, such as alkoxy.

Heteroaryl substituents include compounds with a 5 to 7 member aromatic ring containing one or more heteroatoms, for example from 1 to 4 heteroatoms, selected from N, O and S. Typical heteroaryl substituents include furyl, thienyl, pyrrole, pyrazole, triazole, thiazole, oxazole, pyridine, pyrimidine, isoxazolyl, pyrazine and the like. Unless otherwise noted, heteroaryl substituents are unsubstituted or substituted on a carbon atom by one or more suitable substituents, including alkyl, the alkyl substituents identified above, and another heteroaryl substituent. Nitrogen atoms are unsubstituted or substituted, for example by R₁₃; especially useful N substituents include H, C₁ - C₄ alkyl, acyl, aminoacyl, and sulfonyl.

Arylalkyl substituents include groups of the formula -(CH₂)ₙ₅-aryl, -(CH₂)ₙ₅₋₁-(CH-aryl)-(CH₂)ₙ₅-aryl or -(CH₂)ₙ₅₋₁CH(aryl)(aryl) wherein aryl and n5 are defined above. Such arylalkyl substituents include benzyl, 2-phenylethyl, 1-phenylethyl, tolyl-3-propyl, 2-phenylpropyl, diphenylmethyl, 2-diphenylethyl, 5,5-dimethyl-3-phenylpentyl and the like. Arylalkyl substituents are unsubstituted or substituted in the alkyl moiety or the aryl moiety or both as described above for alkyl and aryl substituents.

Heteroarylalkyl substituents include groups of the formula -(CH₂)ₙ₅-heteroaryl wherein heteroaryl and n5 are defined above and the bridging group is linked to a carbon or a nitrogen of the heteroaryl portion, such as 2-, 3- or 4-pyridylmethyl, imidazolylmethyl, quinolylethyl, and pyrrolylbutyl. Heteroaryl substituents are unsubstituted or substituted as discussed above for heteroaryl and alkyl substituents.

Amino acyl substituents include groups of the formula -C(O)-(CH₂)ₙ-C(H)(NR₁₃R₁₄)-(CH₂)ₙ-R₅ wherein n, R₁₃, R₁₄ and R₅ are described above. Suitable aminoacyl substituents include natural and non-natural amino acids such as glycinyl, D-tryptophanyl, L-lysinyl, D- or L-homoserinyl, 4-aminobutryic acyl, ±-3-amin-4-hexenoyl.

Non-aromatic polycycle substituents include bicyclic and tricyclic fused ring systems where each ring can be 4-9 membered and each ring can contain zero, 1 or more double and/or triple bonds. Suitable examples of non-aromatic polycycles include decalin, octahydroindene, perhydrobenzocycloheptene, perhydrobenzo-[*f*]-azulene. Such substituents are unsubstituted or substituted as described above for cycloalkyl groups.

Mixed aryl and non-aryl polycycle substituents include bicyclic and tricyclic fused ring systems where each ring can be 4 - 9 membered and at least one ring is aromatic. Suitable examples of mixed aryl and non-aryl polycycles include methylenedioxyphenyl, *bis-*methylenedioxyphenyl, 1,2,3,4-tetrahydronaphthalene, dibenzosuberane, dihdydroanthracene, 9H-fluorene. Such substituents are unsubstituted or substituted by nitro or as described above for cycloalkyl groups.

Polyheteroaryl substituents include bicyclic and tricyclic fused ring systems where each ring can independently be 5 or 6 membered and contain one or more heteroatom, for example, 1, 2, 3, or 4 heteroatoms, chosen from O, N or S such that the fused ring system is aromatic. Suitable examples of polyheteroaryl ring systems include quinoline, isoquinoline, pyridopyrazine, pyrrolopyridine, furopyridine, indole, benzofuran, benzothiofuran, benzindole, benzoxazole, pyrroloquinoline, and the like. Unless otherwise noted, polyheteroaryl substituents are unsubstituted or substituted on a carbon atom by one or more suitable substituents, including alkyl, the alkyl substituents identified above and a substituent of the formula -O-(CH₂CH=CH(CH₃)(CH₂))₁₋₃H. Nitrogen atoms are unsubstituted or substituted, for example by R₁₃; especially useful N substituents include H, C₁ - C₄ alkyl, acyl, aminoacyl, and sulfonyl.

Non-aromatic polyheterocyclic substituents include bicyclic and tricyclic fused ring systems where each ring can be 4 - 9 membered, contain one or more heteroatom, for example, 1, 2, 3, or 4 heteroatoms, chosen from O, N or S and contain zero or one or more C-C double or triple bonds. Suitable examples of non-aromatic polyheterocycles include hexitol, cis-perhydro-cyclohepta[b]pyridinyl, decahydro-benzo[f][1,4]oxazepinyl, 2,8-dioxabicyclo[3.3.0]octane, hexahydro-thieno[3,2-b]thiophene, perhydropyrrolo[3,2-b]pyrrole, perhydronaphthyridine, perhydro-1H-dicyclopenta[b,e]pyran. Unless otherwise noted, non-aromatic polyheterocyclic substituents are unsubstituted or substituted on a carbon atom by one or more substituents, including alkyl and the alkyl substituents identified above. Nitrogen atoms are unsubstituted or substituted, for example, by R₁₃; especially useful N substituents include H, C₁ - C₄ alkyl, acyl, aminoacyl, and sulfonyl.

Mixed aryl and non-aryl polyheterocycles substituents include bicyclic and tricyclic fused ring systems where each ring can be 4 - 9 membered, contain one or more heteroatom chosen from O, N or S, and at least one of the rings must be aromatic. Suitable examples of mixed aryl and non-aryl polyheterocycles include 2,3-dihydroindole, 1,2,3,4-tetrahydroquinoline, 5,11-dihydro-10H-dibenz[b,e][1,4]diazepine, 5H-dibenzo[b,e][1,4]diazepine, 1,2-dihydropyrrolo[3,4-b][1,5]benzodiazepine, 1,5-dihydro-pyrido[2,3-b][1,4]diazepin-4-one, 1,2,3,4,6,11-hexahydro-benzo[b]pyrido[2,3-e][1,4]diazepin-5-one. Unless otherwise noted, mixed aryl and non-aryl polyheterocyclic substituents are unsubstituted or substituted on a carbon atom by one or more suitable substituents, including, -N-OH, =N-OH, alkyl and the alkyl substituents identified above. Nitrogen atoms are unsubstituted or substituted, for example, by R₁₃; especially useful N substituents include H, C₁ - C₄ alkyl, acyl, aminoacyl, and sulfonyl.

Amino substituents include primary, secondary and tertiary amines and in salt form, quaternary amines. Examples of amino substituents include mono- and di-alkylamino, mono- and di-aryl amino, mono- and di-arylalkyl amino, aryl-arylalkylamino, alkyl-arylamino, alkyl-arylalkylamino and the like.

Sulfonyl substituents include alkylsulfonyl and arylsulfonyl, for example methane sulfonyl, benzene sulfonyl, tosyl and the like.

Acyl substituents include groups of formula -C(O)-W, -OC(O)-W, -C(O)-O-W or - C(O)NR₁₃R₁₄, where W is R₁₆, H or cycloalkylalkyl.

Acylamino substituents include substituents of the formula -N(R₁₂)C(O)-W, - N(R₁₂)C(O)-O-W, and -N(R₁₂)C(O)-NHOH and R₁₂ and W are defined above.

The R₂ substituent HON-C(O)-CH=C(R₁)-aryl-alkyl- is a group of the formula

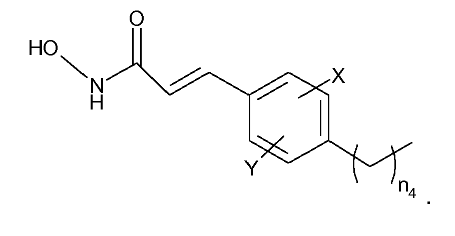

Preferences for each of the substituents include the following:
R₁ is H, halo, or a straight chain C₁-C₄ alkyl;
R₂ is selected from H, C₁-C₆ alkyl, C₄ - C₉ cycloalkyl, C₄ - C₉ heterocycloalkyl, alkylcycloalkyl, aryl, heteroaryl, arylalkyl, heteroarylalkyl, -(CH₂)ₙC(O)R₆, amino acyl, and -(CH₂)ₙR₇;
R₃ and R₄ are the same or different and independently selected from H, and C₁-C₆ alkyl, or R₃ and R₄ together with the carbon to which they are bound represent C=O, C=S, or C=NR₈;
R₅ is selected from H, C₁-C₆ alkyl, C₄ - C₉ cycloalkyl, C₄ - C₉ heterocycloalkyl, aryl, heteroaryl, arylalkyl, heteroarylalkyl, a aromatic polycycle, a non-aromatic polycycle, a mixed aryl and non-aryl polycycle, polyheteroaryl, a non-aromatic polyheterocycle, and a mixed aryl and non-aryl polyheterocycle;
n, n₁, n₂ and n₃ are the same or different and independently selected from 0 - 6, when n₁ is 1-6, each carbon atom is unsubstituted or independently substituted with R₃ and/or R₄;
X and Y are the same or different and independently selected from H, halo, C₁-C₄ alkyl, CF₃, NO₂, C(O)R₁, OR₉, SR₉, CN, and NR₁₀R₁₁;
R₆ is selected from H, C₁-C₆ alkyl, C₄ - C₉ cycloalkyl, C₄ - C₉ heterocycloalkyl, alkylcycloalkyl, aryl, heteroaryl, arylalkyl, heteroarylalkyl, OR₁₂, and NR₁₃R₁₄;
R₇ is selected from OR₁₅, SR₁₅, S(O)R₁₆, SO₂R₁₇, NR₁₃R₁₄, and NR₁₂SO₂R₆;
R₈ is selected from H, OR₁₅, NR₁₃R₁₄, C₁-C₆ alkyl, C₄ - Cg cycloalkyl, C₄ - C₉ heterocycloalkyl, aryl, heteroaryl, arylalkyl, and heteroarylalkyl;
Rg is selected from C₁ - C₄ alkyl and C(O)-alkyl;
R₁₀ and R₁₁ are the same or different and independently selected from H, C₁-C₄ alkyl, and -C(O)-alkyl;
R₁₂ is selected from H, C₁-C₆ alkyl, C₄ - Cg cycloalkyl, C₄ - Cg heterocycloalkyl, aryl, heteroaryl, arylalkyl, and heteroarylalkyl;
R₁₃ and R₁₄ are the same or different and independently selected from H, C₁-C₆ alkyl, C₄ - Cg cycloalkyl, C₄ - Cg heterocycloalkyl, aryl, heteroaryl, arylalkyl, heteroarylalkyl and amino acyl;
R₁₅ is selected from H, C₁-C₆ alkyl, C₄ - Cg cycloalkyl, C₄ - Cg heterocycloalkyl, aryl, heteroaryl, arylalkyl, heteroarylalkyl and (CH₂)ₘZR₁₂;
R₁₆ is selected from C₁-C₆ alkyl, C₄ - Cg cycloalkyl, C₄ - Cg heterocycloalkyl, aryl, heteroaryl, arylalkyl, heteroarylalkyl and (CH₂)ₘZR₁₂;
R₁₇ is selected from C₁-C₆ alkyl, C₄ - Cg cycloalkyl, C₄ - Cg heterocycloalkyl, aryl, heteroaryl, arylalkyl, heteroarylalkyl and NR₁₃R₁₄;
m is an integer selected from 0 to 6; and Z is selected from O, NR₁₃, S, S(O), or a pharmaceutically acceptable salt thereof.

Useful compounds of the formula (I) include those wherein each of R₁, X, Y, R₃, and R₄ is H, including those wherein one of n₂ and n₃ is zero and the other is 1, especially those wherein R₂ is H or -CH₂-CH₂-OH.

One suitable genus of hydroxamate compounds are those of formula Xa: 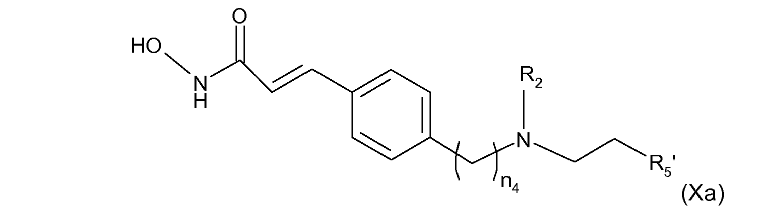 wherein
n₄ is 0-3,
R₂ is selected from H, C₁-C₆ alkyl, C₄ - C₉ cycloalkyl, C₄ - C₉ heterocycloalkyl, alkylcycloalkyl, aryl, heteroaryl, arylalkyl, heteroarylalkyl, -(CH₂)ₙC(O)R₆, amino acyl and -(CH₂)ₙR₇;
R₅' is heteroaryl, heteroarylalkyl (e.g., pyridylmethyl), aromatic polycycles, non-aromatic polycycles, mixed aryl and non-aryl polycycles, polyheteroaryl, or mixed aryl and non-aryl polyheterocycles,
or a pharmaceutically acceptable salt thereof

Another suitable genus of hydroxamate compounds are those of formula Xa, wherein
n₄ is 0-3,
R₂ is selected from H, C₁-C₆ alkyl, C₄ - C₉ cycloalkyl, C₄ - C₉ heterocycloalkyl, alkylcycloalkyl, aryl, heteroaryl, arylalkyl, heteroarylalkyl, -(CH₂)ₙC(O)R₆, amino acyl and -(CH₂)ₙR₇;
R₅' is aryl, arylalkyl, aromatic polycycles, non-aromatic polycycles, and mixed aryl and non-aryl polycycles; especially aryl, such as p-fluorophenyl, p-chlorophenyl, p-O-C₁- C₄-alkylphenyl, such as p-methoxyphenyl, and p-C₁-C4-alkylphenyl; and arylalkyl, such as benzyl, *ortho, meta or para*-fluorobenzyl, *ortho, meta or para*-chlorobenzyl, *ortho, meta or para*-mono, di or tri-O-C₁-C₄-alkylbenzyl, such as *ortho, meta or para-* methoxybenzyl, *m,p*-diethoxybenzyl, *o,m,p*-triimethoxybenzyl , and *ortho, meta or para-* mono, di or tri C₁-C₄-alkylphenyl, such as *p*-methyl, *m,m*-diethylphenyl,
or a pharmaceutically acceptable salt thereof.

Another interesting genus is the compounds of formula Xb: 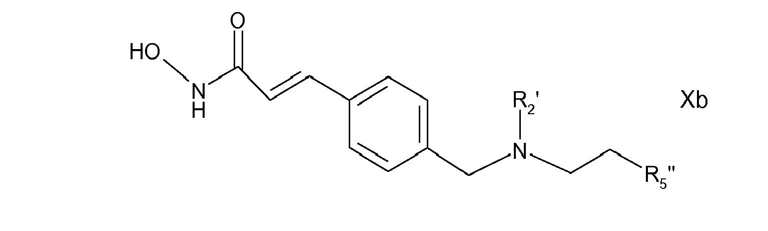 wherein
R₂' is selected from H, C₁-C₆ alkyl, C₄-C₆ cycloalkyl, cycloalkylalkyl (e.g., cyclopropylmethyl), (CH₂)₂₋₄OR₂₁ where R₂₁ is H, methyl, ethyl, propyl, and *i*-propyl, and
R₅" is unsubstituted 1*H*-indol-3-yl, benzofuran-3-yl or quinolin-3-yl, or substituted 1*H*-indol- 3-yl, such as 5-fluoro-1*H*-indol-3-yl or 5-methoxy-1*H*-indol-3-yl, benzofuran-3-yl or quinolin- 3-yl,
or a pharmaceutically acceptable salt thereof.

Another interesting genus of hydroxamate compounds are the compounds of formula Xc: 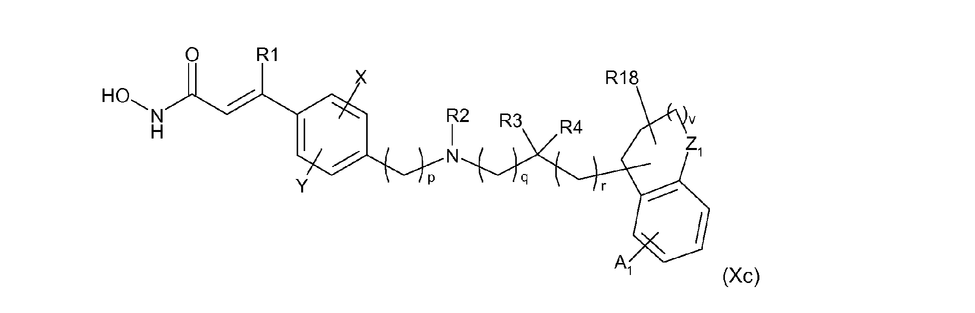 wherein
the ring containing Z₁ is aromatic or non-aromatic, which non-aromatic rings are saturated or unsaturated,
Z₁ is O, S or N-R₂₀,
R18 is H, halo, C₁-C₆alkyl (methyl, ethyl, t-butyl), C₃-C₇cycloalkyl, aryl, for example unsubstituted phenyl or phenyl substituted by 4-OCH₃ or 4-CF₃, or heteroaryl, such as 2-furanyl, 2-thiophenyl or 2-, 3- or 4-pyridyl;
R₂₀ is H, C₁-C₆alkyl, C₁-C₆alkyl-C₃-C₉cycloalkyl (e.g., cyclopropylmethyl), aryl, heteroaryl, arylalkyl (e.g., benzyl), heteroarylalkyl (e.g., pyridylmethyl), acyl (acetyl, propionyl, benzoyl) or sulfonyl (methanesulfonyl, ethanesulfonyl, benzenesulfonyl, toluenesulfonyl)
A₁ is 1, 2 or 3 substituents which are independently H, C₁-C-₆alkyl, -OR₁₉, halo, alkylamino, aminoalkyl, halo, or heteroarylalkyl (e.g., pyridylmethyl),
R₁₉ is selected from H, C₁-C₆alkyl, C₄-C₉cycloalkyl, C₄-C₉heterocycloalkyl, aryl, heteroaryl, arylalkyl (e.g., benzyl), heteroarylalkyl (e.g., pyridylmethyl) and -(CH₂CH=CH(CH₃)(CH2₎)₁₋₃H;
R₂ is selected from H, C₁-C₆ alkyl, C₄ - C₉ cycloalkyl, C₄ - C₉ heterocycloalkyl, alkylcycloalkyl, aryl, heteroaryl, arylalkyl, heteroarylalkyl, -(CH₂)ₙC(O)R₆, amino acyl and -(CH₂)ₙR₇;
v is 0, 1 or 2,
p is 0-3, and
q is 1-5 and r is 0 or
q is 0 and r is 1-5,
or a pharmaceutically acceptable salt thereof. The other variable substituents are as defined above.

Especially useful compounds of formula (Xc) are those wherein R₂ is H, or - (CH₂)ₚCH₂OH, wherein p is 1-3, especially those wherein R₁ is H; such as those wherein R₁ is H and X and Y are each H, and wherein q is 1-3 and r is 0 or wherein q is 0 and r is 1-3, especially those wherein Z₁ is N-R₂₀. Among these compounds R₂ is preferably H or -CH₂-CH₂-OH and the sum of q and r is preferably 1.

Another interesting genus of hydroxamate compounds are the compounds of formula (Xd) 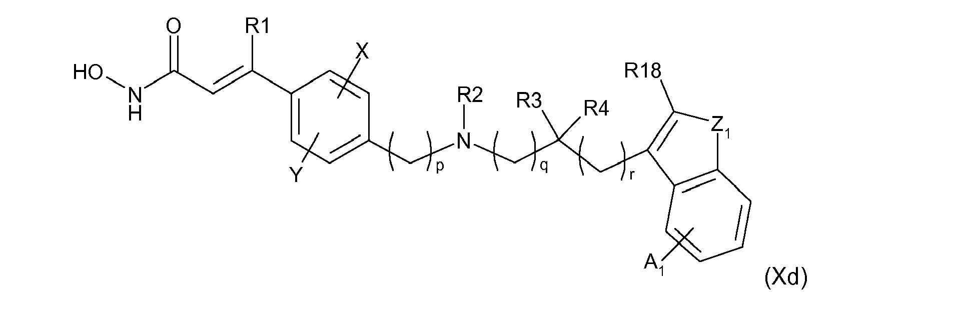 wherein
Z₁ is O, S or N-R₂₀,
R18 is H, halo, C₁-C₆alkyl (methyl, ethyl, t-butyl), C₃-C₇cycloalkyl, aryl, for example, unsubstituted phenyl or phenyl substituted by 4-OCH₃ or 4-CF₃, or heteroaryl,
R₂₀ is H, C₁-C₆alkyl, C₁-C₆alkyl-C₃-C₉cycloalkyl (e.g., cyclopropylmethyl), aryl, heteroaryl, arylalkyl (e.g., benzyl), heteroarylalkyl (e.g., pyridylmethyl), acyl (acetyl, propionyl, benzoyl) or sulfonyl (methanesulfonyl, ethanesulfonyl, benzenesulfonyl, toluenesulfonyl),
A₁ is 1, 2 or 3 substituents which are independently H, C₁-C-₆alkyl, -OR₁₉, or halo,
R₁₉ is selected from H, C₁-C₆alkyl, C₄-C₉cycloalkyl, C₄-C₉heterocycloalkyl, aryl, heteroaryl, arylalkyl (e.g., benzyl), and heteroarylalkyl (e.g., pyridylmethyl);
p is 0-3, and
q is 1-5 and r is 0 or
q is 0 and r is 1-5,
or a pharmaceutically acceptable salt thereof. The other variable substituents are as defined above.

Especially useful compounds of formula (Xd) are those wherein R₂ is H, or - (CH₂)ₚCH₂OH, wherein p is 1-3, especially those wherein R₁ is H; such as those wherein R₁ is H and X and Y are each H, and wherein q is 1-3 and r is 0 or wherein q is 0 and r is 1-3.

Among these compounds R₂ is preferably H or -CH₂-CH₂-OH and the sum of q and r is preferably 1.

The present invention further relates to compounds of the formula (Xe) 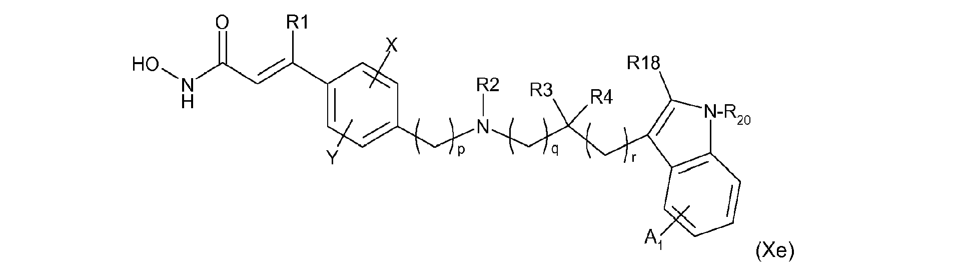 or a pharmaceutically acceptable salt thereof. The variable substituents are as defined above.

Especially useful compounds of formula (Xe) are those wherein R18 is H, fluoro, chloro, bromo, a C₁-C₄alkyl group, a substituted C₁-C₄alkyl group, a C₃-C₇cycloalkyl group, unsubstituted phenyl, phenyl substituted in the para position, or a heteroaryl (e.g., pyridyl) ring.

Another group of useful compounds of formula Xe are those wherein R₂ is H, or - (CH₂)ₚCH₂OH, wherein p is 1-3, especially those wherein R₁ is H; such as those wherein R₁ is H and X and Y are each H, and wherein q is 1-3 and r is 0 or wherein q is 0 and r is 1-3. Among these compounds R₂ is preferably H or -CH₂-CH₂-OH and the sum of q and r is preferably 1. Among these compounds p is preferably 1 and R3 and R4 are preferably H.

Another group of useful compounds of formula (le) are those wherein R18 is H, methyl, ethyl, t-butyl, trifluoromethyl, cyclohexyl, phenyl, 4-methoxyphenyl, 4-trifluoromethylphenyl, 2-furanyl, 2-thiophenyl, or 2-, 3- or 4-pyridyl wherein the 2-furanyl, 2-thiophenyl and 2-, 3- or 4-pyridyl substituents are unsubstituted or substituted as described above for heteroaryl rings; R₂ is H, or-(CH₂)ₚCH₂OH, wherein p is 1-3; especially those wherein R₁ is H and X and Y are each H, and wherein q is 1-3 and r is 0 or wherein q is 0 and r is 1-3. Among these compounds R₂ is preferably H or-CH₂-CH₂-OH and the sum of q and r is preferably 1.

Those compounds of formula Xe wherein R₂₀ is H or C₁-C₆alkyl, especially H, are important members of each of the subgenuses of compounds of formula Xe described above.

N-hydroxy-3-[4-[[(2-hydroxyethyl)[2-(1H-indol-3-yl)ethyl]-amino]methyl]phenyl]-2E-2-propenamide, N-hydroxy-3-[4-[[[2-(1H-indol-3-yl)ethyl]-amino]methyl]phenyl]-2E-2-propenamide and *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof, are important compounds of formula (le).

The present invention further relates to the compounds of the formula (Xf): 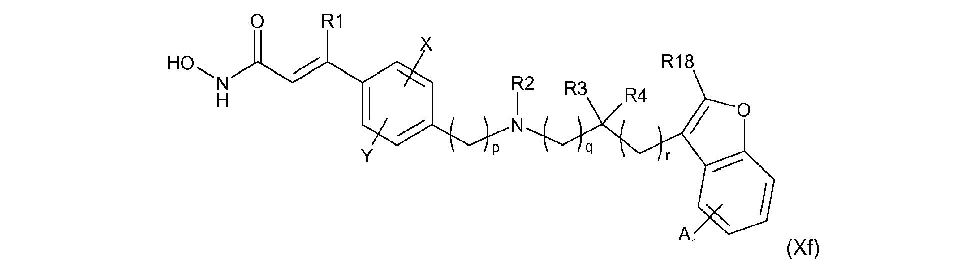 or a pharmaceutically acceptable salt thereof. The variable substituents are as defined above.

Useful compounds of formula (If) are include those wherein R₂ is H, or -(CH₂)ₚCH₂OH, wherein p is 1-3, especially those wherein R₁ is H; such as those wherein R₁ is H and X and Y are each H, and wherein q is 1-3 and r is 0 or wherein q is 0 and r is 1-3. Among these compounds R₂ is preferably H or -CH₂-CH₂-OH and the sum of q and r is preferably 1.

N-hydroxy-3-[4-[[[2-(benzofur-3-yl)-ethyl]-amino]methyl]phenyl]-2E-2-propenamide,or a pharmaceutically acceptable salt thereof, is an important compound of formula (Xf).

The synthesis of the histone deacetylase inhibitors of the invention, i.e. the HDAI compounds described above and to be used in combination with the FLT-3 kinase inhibitors mentioned hereinbefore, can be prepared as generally and specifically disclosed in EP 1 318 980 and WO 02/22577, the entire contents of which being herewith incorporated by reference.

HDAI compounds used in the combination of the present invention are typically those which have an IC₅₀ of less than 2 µM, especially of less than 500 nM, and most preferably of less than 100 nM in the histone deacetylase inhibition assay described in Example B2 of WO 02/22577.

The present invention in particular provides a method of treating myelodysplastic syndromes, lymphomas and leukemias, in particular acute myeloid leukemia (AML), and also solid tumors such as e.g. colorectal cancer (CRC) and non-small cell lung cancer (NSCLC), comprising administering to a mammalin need of such a treatment a therapeutically effective amount of a combination of a FLT-3 kinase inhibitor and a histone deacetylase inhibitor (HDAI), each in free form or in form of a pharmaceutically acceptable salt or prodrug, respectively.

Preferably the instant invention provides a method for treating mammals, especially humans, suffering from myelodysplastic syndromes, lymphomas and leukemias, in particular acute myeloid leukemia (AML), and also solid tumors such as e.g. colorectal cancer (CRC) and non-small cell lung cancer (NSCLC) comprising administering to a mammal in need of such treatment an therapeutically effective amount of a combination of *N*-[(9*S*,10*R*,11*R*,13*R*)-2,3,10,11,12,13-hexahydro-10-methoxy-9-methyl-1-oxo-9,13-epoxy-1*H*,9*H*-diindolo[1,2,3-gh:3',2',1'-Im]pyrrolo[3,4-j][1,7]benzodiazonin-11-yl]-*N-*methylbenzamide of the formula (VII), or a pharmaceutically acceptable salt thereof and N-hydroxy-3-[4-[[[2-(2-methyl-1*H*-indol-3-yl)-ethyl]-amino]methyl]phenyl]-2*E*-2-propenamide, or a pharmaceutically acceptable salt or prodrug thereof or N-hydroxy-3-[4-[[(2-hydroxyethyl)[2-(1H-indol-3-yl)ethyl]-amino]methyl]phenyl]-2E-2-propenamide or a pharmaceutically acceptable salt or prodrug thereof.

In another embodiment, the instant invention relates to the use of a combination of a FLT-3 kinase inhibitor and a histone deacetylase inhibitor (HDAI), each in free form or in form of a pharmaceutically acceptable salt or prodrug, respectively, for treating myelodysplastic syndromes, lymphomas and leukemias, in particular acute myeloid leukemia (AML), and also solid tumors such as e.g. colorectal cancer (CRC) and non-small cell lung cancer (NSCLC).

In a further embodiment, the instant invention relates to the use of a combination of a FLT-3 kinase inhibitor and a histone deacetylase inhibitor (HDAI), each in free form or in form of a pharmaceutically acceptable salt or prodrug, respectively, for the preparation of a pharmaceutical composition for treating myelodysplastic syndromes, lymphomas and leukemias, in particular acute myeloid leukemia (AML), and also solid tumors such as e.g. colorectal cancer (CRC) and non-small cell lung cancer (NSCLC).

According to the invention a combination of *N*-[(9*S*,10*R*,11*R*,13*R*)-2,3,10,11,12,13-hexahydro-10-methoxy-9-methyl-1-oxo-9,13-epoxy-1*H*,9*H*-diindolo[1,2,3-gh:3',2',1'-Im]pyrrolo[3,4-j][1,7]benzodiazonin-11-yl]-*N*-methylbenzamide of the formula (VII), or a pharmaceutically acceptable salt thereof and either *N*-hydroxy-3-[4-[[[2-(2-methyl-1*H*-indol-3-yl)-ethyl]-amino]methyl]phenyl]-2*E*-2-propenamide, or a pharmaceutically acceptable salt or prodrug thereof or N-hydroxy-3-[4-[[(2-hydroxyethyl)[2-(1H-indol-3-yl)ethyl]-amino]methyl]phenyl]-2E-2-propenamide or a pharmaceutically acceptable salt or prodrug thereof are the preferred combinations of a FLT-3 kinase inhibitor and a histone deacetylase inhibitor (HDAI).

The combination of a FLT-3 kinase inhibitor and a histone deacetylase inhibitor (HDAI), each in free form or in form of a pharmaceutically acceptable salt or prodrug, respectively, for treating myelodysplastic syndromes, lymphomas and leukemias, in particular acute myeloid leukemia (AML), and also solid tumors such as e.g. colorectal cancer (CRC) and non-small cell lung cancer (NSCLC) may be a free or fixed combination of the combination partners.

In one aspect, the present invention also relates to a combination, such as a combined preparation or a pharmaceutical composition, which comprises (a) a FLT-3 inhibitor, especially the FLT-3 inhibitors specifically mentioned hereinbefore, in particular those mentioned as being preferred, and (b) an HDAI, especially the HDAls mentioned hereinbefore, in particular those mentioned as being preferred, in which the active ingredients (a) and (b) are present in each case in free form or in the form of a pharmaceutically acceptable salt, for simultaneous, concurrent, separate or sequential use.

The term "a combined preparation" defines especially a "kit of parts" in the sense that the combination partners (a) and (b) as defined above can be dosed independently or by use of different fixed combinations with distinguished amounts of the combination partners (a) and (b), i.e., simultaneously, concurrently, separately or sequentially. The parts of the kit of parts can then, e.g., be administered simultaneously or chronologically staggered, that is at different time points and with equal or different time intervals for any part of the kit of parts. The ratio of the total amounts of the combination partner (a) to the combination partner (b) to be administered in the combined preparation can be varied, e.g. in order to cope with the needs of a patient sub-population to be treated or the needs of the single patient which different needs can be due to the particular disease, severity of the disease, age, sex, body weight, etc. of the patients.

Aas mentioned above the precise dosage of the FLT-3 inhibitor and the HDAI to be employed for treating the diseasesand conditions mentioned hereinbefore depends upon several factors including the host, the nature and the severity of the condition being treated, the mode of administration. However, in general, satisfactory results are achieved when the FLT-3 inhibitor is administered parenterally, e.g., intraperitoneally, intravenously, intramuscularly, subcutaneously, intratumorally, or rectally, or enterally, e.g., orally, preferably intravenously or, preferably orally, intravenously at a daily dosage of 0.1 to 10 mg/kg body weight, preferably 1 to 5 mg/kg body weight. In human trials a total dose of 225 mg/day was most presumably the Maximum Tolerated Dose (MTD). A preferred intravenous daily dosage is 0.1to 10 mg/kg body weight or, for most larger primates, a daily dosage of 200-300 mg. A typical intravenous dosage is 3 to 5 mg/kg, three to five times a week.

Most preferably, the FLT-3 inhibitors, especially MIDOSTAURIN, are administered orally, by dosage forms such as microemulsions, soft gels or solid dispersions in dosages up to about 250 mg/day, in particular 225 mg/day, administered once, twice or three times daily.

Usually, a small dose is administered initially and the dosage is gradually increased until the optimal dosage for the host under treatment is determined. The upper limit of dosage is that imposed by side effects and can be determined by trial for the host being treated.

The FLT-3 inhibitors and the HDAI compounds may be combined with one or more pharmaceutically acceptable carriers and, optionally, one or more other conventional pharmaceutical adjuvants and administered enterally, e.g. orally, in the form of tablets, capsules, caplets, etc. or parenterally, e.g., intraperitoneally or intravenously, in the form of sterile injectable solutions or suspensions. The enteral and parenteral compositions may be prepared by conventional means.

The infusion solutions according to the present invention are preferably sterile. This may be readily accomplished, e.g. by filtration through sterile filtration membranes. Aseptic formation of any composition in liquid form, the aseptic filling of vials and/or combining a pharmaceutical composition of the present invention with a suitable diluent under aseptic conditions are well known to the skilled addressee.

The FLT-3 inhibitors and HDAI compounds may be formulated into enteral and parenteral pharmaceutical compositions containing an amount of the active substance that is effective for treating the diseases and conditions named hereinbefore, such compositions in unit dosage form and such compositions comprising a pharmaceutically acceptable carrier.

Examples of useful compositions of FLT-3 inhibitors are described in the European patents No. 0 296 110, No. 0 657 164, No. 0 296 110, No.0 733 372, No.0 711 556, No.0 711 557.

The preferred compositions of FLT-3 inhibitors are described in the European patent No. 0 657 164 published on June 14, 1995. The described pharmaceutical compositions comprise a solution or dispersion of compounds of formula I such as MIDOSTAURIN in a saturated polyalkylene glycol glyceride, in which the glycol glyceride is a mixture of glyceryl and polyethylene glycol esters of one or more C8-C18 saturated fatty acids.

Two manufacture processes of such compositions of FLT-3 inhibitors are described hereafter.

### Composition A:

Gelucire 44/14 (82 parts) is melted by heating to 60° C. Powdered MIDOSTAURIN (18 parts) is added to the molten material. The resulting mixture is homogenised and the dispersion obtained is introduced into hard gelatin capsules of different size, so that some contain a 25mg dosage and others a 75mg dosage of the MIDOSTAURIN. The resulting capsules are suitable for oral administration.

### Composition B:

Gelucire 44/14 (86 parts) is melted by heating to 60° C. Powdered MIDOSTAURIN (14 parts) is added to the molten material. The mixture is homogenised and the dispersion obtained is introduced into hard gelatin capsules of different size, so that some contain a 25mg dosage and others a 75mg dosage of the MIDOSTAURIN. The resulting capsules are suitable for oral administration.

Gelucire 44/14 available commercially from Gattefossé; is a mixture of esters of C8-C18 saturated fatty acids with glycerol and a polyethylene glycol having a molecular weight of about 1500, the specifications for the composition of the fatty acid component being, by weight, 4-10% caprylic acid, 3-9% capric acid, 40-50% lauric acid, 14-24% myristic acid, 4-14% palmitic acid and 5-15% stearic acid.

A preferred example of Gelucire formulation consists of:
Gelucire (44/14): 47 g
MIDOSTAURIN: 3.0g filled into a 60 mL Twist off flask

A preferred example of soft gel will contain the following Microemulsion:

| | |
|---|---|
| Cornoil glycerides | 85.0 mg |
| Polyethylenglykol 400 | 128.25 mg |
| Cremophor RH 40 | 213.75 mg |
| MIDOSTAURIN | 25.0 mg |
| DL alpha Tocopherol | 0.5 mg |
| Ethanol absolute | 33.9 mg |
| Total | 486.4 mg |

However, it should be clearly understood that it is for purposes of illustration only.

It can be shown by the test methods described below that the combination of a FLT-3 inhibitor and a HDAI compound are more effective than treatment with either of the agents alone. In these studies determined the cell cycle effects and apoptosis induced by a histone deacetylase inhibitor, preferably a cinnamyl hydroxamate, and a FLT-3 kinase inhibitor, preferably 4-benzyl staurosporine, against human AML cells that either express the constitutively active mutant or wild type FLT-3 tyrosine kinase are demostrated.

A newly developed flow cytometry (FCM) assay, utilizing anti-FLT-3 or phospho (p)-FLT-3 antibody, is used to demonstrate that while MV4-11 (MV) cells express both FLT-3 and p-FLT-3, RS4-11 (RS) cells only express FLT-3 on their cell surface. Exposure to 20 to 200 nM of a preferred FLT-3 inhibitor induces cell cycle G1 phase accumulation and, in a dose-dependent manner, significantly more apoptosis of MV than RS cells. This is associated with marked attenuation of p-FLT-3, p-AKT and p-ERK1/2 but not of FLT-3, AKT or ERK1/2 levels, as determined by Western analyses. The preferred FLT-3 inhibitor also inhibits the surface expression of p-FLT-3 but not of FLT-3 (as can be determined by FCM) on MV cells. In contrast to the preferred FLT-3 inhibitor, treatment with a preferred HDAI compound attenuates both FLT-3 and p-FLT-3 levels in a dose-dependent manner in MV and RS cells, as can be determined both by Western and FCM analyses. Exposure to a preferred HDAI compound (20 to 100 nM) also down regulates the levels of p-FLT-3, p-AKT and p-ERK1/2. Significantly, co-treatment with a preferred FLT-3 inhibitor and a preferred HDAI compound surprinsingly induces apoptosis of MV and RS cells. This is associated with more attenuation of p-FLT-3, p-AKT and p-ERK1/2 in MV cells.

Preferably, there is at least one beneficial effect, e.g., a mutual enhancing of the effect of the first and second active ingredient, in particular a synergism, e.g. a more than additive effect, additional advantageous effects, less side effects, a combined therapeutical effect in a otherwise non-effective dosage of one or both of the first and second active ingredient, and especially a strong synergism the active ingredients.

The molar ratio of FLT-3 inhibitor/HDAI compound in the combination is generally from 1/10 to 10/1, preferably from 1/5 to 5/1, e.g. ½, 1 /1, 2/1, or 3/1.

In three samples of primary leukemia blasts with high p-FLT-3 expression from patients with AML in relapse, the combined treatment with a preferred FLT-3 inhibitor and a preferred HDAI compound again induces more apoptosis and attenuation of p-FLT-3 levels in a more synergystic, preferably additive way. In conclusion, this clearly demonstrates for the first time that a) the combination of a preferred FLT-3 inhibitor and a preferred HDAI compound is highly effective in attenuating p-FLT-3, p-AKT and p-ERK1/2 and in inducing apoptosis of human AML cells with the constitutively active FLT-3 tyrosine kinase, and b) an FCM-based assay may be useful in distinguishing AML with constitutively higher cell surface expression of p-FLT-3 and FLT-3, as well as in assessing the response to inhibitors of p-FLT-3 kinase in AML cells.

The simultaneous measurement of surface FLT-3 kinase and Phospho (P Y591) FLT-3 kinase in Acute Leukemic cells using a flow cytometric assay can be carried out as follows:

Leukemic cells are harvested by spinning at 1000 rpm at 4 deg for 5 minutes. The cells are washed twice with cold phosphate-buffered saline (PBS) (1 X). Equal numbers of cells are utilized for FLT-3 and p-FLT-3 analyses.

For surface FLT-3 expression, the cells are incubated on ice for thirty minutes in PBS (1 X) containing 3% fetal bovine serum (FBS) (blocking buffer). Subsequently, the cells are washed twice with cold PBS (1 X). Cells are then incubated with either 0.2 µg of anti-FLT-3 antibody (sc-19635, Santa Cruz Biotechnology, CA) or concentration-matched isotype, control antibody (IgG1, Caltag, Burlingame, CA) diluted in the blocking buffer and kept on ice for one hour. Cells are then washed twice in PBS (1 X) and incubated in FITC-conjugated secondary antibody (Molecular Probes, Eugene, OR) for additional thirty minutes on ice. The cells are then rinsed twice with PBS (1 X) and re suspended in 400 µL PBS (1 X) The fluorescence is measured by FACScan Cytometer (San Jose, CA).

To determine the p-FLT-3 expression, leukemia cells are fixed and permeabilized. Cells are fixed in 1% formaldehyde at 37 degrees for ten minutes, followed by incubation on ice for ten minutes. Cells are then spun down and permeabilized by resuspending them in ice cold 90% methanol for thirty minutes. Following this, cells are washed twice in the blocking buffer (PBS (1 X) containing 0.5% BSA) and then incubated in the blocking buffer for an additional ten minutes at room temperature (RT). Next, to the cells, either 0.4 µg of monoclonal antibody to p- FLT-3 (Cell Signaling, Beverley, MA) or isotype control antibody (IgG2b, Caltag, Burlingame, CA) is added and cells are then incubated at room temperature for thirty minutes. Cells are then rinsed twice in the blocking buffer, followed by incubation with the FITC-conjugated secondary antibody (Molecular Probes, Eugene, OR). After thirty minutes of incubation, cells are washed twice with PBS (1 X) and resuspended in 400 µl of PBS (1 X) and analyzed by FACScan .

### Example 1

### STUDY DESIGN:

**Reagents:** LAQ824 and PKC412 were provided by Novartis Pharmaceuticals Inc. (East Hanover, NJ). Antibodies for the immunoblot analyses were purchased, as follows: FLT-3, STAT5 and c-Myc from Santa Cruz Biotechnology Inc. (Santa Cruz, CA); p-FLT-3 and p-ERK1/2 from Cell Signaling Technology (Beverly, MA); p-STAT5 from Upstate Biotechnology, Inc. (Lake Placid, NY) and Oncostatin M from R & D Systems Inc. (Minneapolis, MN). The source of the other antibodies used in these studies has been previously described.
**Cells:** Acute leukemia MV4-11 (containing a 30 base pair long ITD in the exon 14 of FLT-3) and RS4-11 (containing wild-type FLT-3) cells were obtained from American Tissue Culture Collection (Manassas, VA) and maintained in culture as previously described. Primary leukemia blasts from four patients with AML in relapse were harvested and purified, as previously described, a protocol study sanctioned by the local institutional review board (IRB).
**Flow cytometry for Cell cycle status and apoptosis assessment:** Flowcytometric evaluation of the cell cycle status and sub-G1 apoptotic population of cells was performed.
**Assessment of % Non-viable and apoptotic cells:** Primary AML cells were stained with trypan blue (Sigma, St. Louis, MO). Number of non-viable cells were determined by counting the cells that showed trypan blue uptake in a hemocytometer, and reported as % of untreated control cells. The % of apoptotic cells were determined by flow cytometry.
**Western Blot Analysis:** Western analyses of proteins from untreated and drug-treated cells were performed.
**Autophosphorylation of FLT-3:** Following incubation with anti-FLT-3 antibody, Protein G agarose beads were washed and incubated with the cell lysates of untreated or drug-treated cells, as previously described. The immunoprecipitates were washed, the proteins were eluted with the SDS sample loading buffer, and following SDS-PAGE immunoblotted with antiphosphotyrosine antibody (PharMingen, San Diego, CA)
**RT-PCR assay for FLT-3 mRNA levels:** RT-PCR analysis was performed, as previously described. To detect FLT-3 ITD, the primer sequences were as follows: forward primer: 5'-TGT CGA GCA GTA CTC TAA ACA-3', reverse primer: 5'-ATC CTA GTA CCT TCC CAA ACT C-3'. For β-actin, the primer sequences were: forward primer: 5'-CTA CAA TGA GCT GCG TGT GG-3' and reverse primer: 5'-AAG GAA GGC TGG AAG AGT GC-3'. The size of the amplified products was 395 bases pairs for the FLT-3 and 527 base pairs for β-actin product, respectively.
**Electrophoretic Mobility Shift Assay (EMSA) for STAT5a:** Untreated or LAQ824 and/or PKC412 treated cells were lysed, nuclear extracts were obtained and the EMSA for the DNA binding activity of STAT5a was performed.
**RESULTS AND DISCUSSION:** Exposure to 10.0 to 50 nM LAQ824 for 48 hours induced a dose-dependent increase in apoptosis of MV4-11 more than RS4-11 cells, along with greater induction of PARP cleavage activity of caspase-3. LAQ824 treatment increased the percentage of MV4-11 (more than RS4-11 cells) in the G1 phase of the cell cycle, which was accompanied by increase in the sub-diploid apoptotic population of cells (p<0.01) (data not shown). LAQ824 treatment induced p21 but attenuated p-FLT-3 and FLT-3 expression in MV4-11 and FLT-3 levels in RS4-11 cells LAQ824 mediated decline in the p-FLT-3 levels was most likely due to inhibition of its auto-phosphorylation, as shown in Table 1

**TABLE 1**

| | | | | **% Non-viable Cells** | | | | |
|---|---|---|---|---|---|---|---|---|
| **Patient** | **Contr** | **PKC-412** | | **LAQ824** | | | **LAQ824 (20 nM) PKC-412** | |
| | | **100** | **500** | **20** | **50 nM** | **250** | **100** | **500** |
| **1** | **12.9** | **27.1** | **64.9** | **21.6** | **66.5** | **98.0** | **59.6** | **73.2** |
| **2** | **11.1** | **23.3** | **41.1** | **21.1** | **51.8** | **99.0** | **50.5** | **64.3** |
| **3** | **10.2** | **19.4** | **24.0** | **37.1** | **46.2** | **54.6** | **33.7** | **35.4** |
| **4** | **10.0** | **9.9** | **10.1** | **10.5** | **26.7** | **32.7** | **12.6** | **16.6** |

Recently FLT-3, and especially p-FLT-3, has been shown to have a chaperone association, as a client protein, with hsp90, and inhibitors of hsp90 disrupt this association, directing FLT-3 to polyubiquitylation and proteasomal degredation. Furthermore, our recent findings have demonstrated that treatment with LAQ824 in addition to causing acetylation of histones H3 and H4 also causes acetylation of hsp90, which inhibits its chaperone function and promotes the proteasomal degredation of its client protein, especially if they have a mutant conformation, e.g., Bcr-Abl. Consistent with these reports, co-treatment with the proteasome inhibitor PS-341 restored LAQ824 mediated attenuation of p-FLT-3 and FLT-3 in MV4-11 cells. Additionally, treatment with up to 50 nM of LAQ824 did not inhibit the mRNA transcript levels of FLT-3 in MV4-11 cells, ruling out the possibility that transcriptional downregulation is a significant contributory mechanism toward LAQ824 mediated repression of FLT-3 in MV4-11 cells. It is also noteworthy that co-treatment with zVAD-fmk, a pan-caspase inhibitor, did not restore the levels of FLT-3 reduced by LAQ824 treatment, making it unlikely that the decline was due to FLT-3 processing by caspases induced by treatment with LAQ824.

Recent studies have shown that FLT-3 activity induces STAT-5 phosphorylation and transactivation of several genes that confer proliferative and/or survival advantage, notably c-Myc, oncostatin M and Pim-2. This function may also be bolstered by the downstream phosphorylation and activity of ERK1/2 and AKT. Consistent with this, LAQ824 mediated down regulation of FLT-3 was associated with attenuation of the levels of p-STAT5 and pAKT in MV4-11 and RS4-11 cells, while pERK1/2 levels were inhibited only in MV4-11 cells. Inhibition of p-STAT5 by LAQ824 was accompanied by attenuation of its DNA binding activity. This was associated with down regulation of c-Myc and oncostatin M levels in MV4-11 and RS4-11 cells. We next determined whether co-treatment with LAQ824 would sensitize MV4-11 cells to apoptosis induced by PKC412. It is shown that co-treatment with 10 or 20 nM LAQ824 enhances apoptosis induced by 20 or 100 nM of PKC412. As compared with either agent alone, co-treatment with LAQ824 (10 nM) and PKC412 (100 nM) was associated with a marked decline in the expression of p-FLT-3, FLT-3, p-STAT5, p-AKT, pERK1/2 and c-Myc, as well as increased processing of PARP. In addition, combined treatment with LAQ824 and PKC412 also inhibited the DNA binding activity of STAT5a more than with either agent alone, a result consistent with the greater decline in the p-FLT-3 levels due to the combination.

We next determined whether the combination of LAQ824 and PKC412 would also have superior activity against primary AML cells isolated from the peripheral blood or bone marrow samples from four patients with AML in relapse. Although not shown, Sample #1 cells contained a duplication of a 51 base pair sequence from base pair 1837 to 1887 and sample #2 cells contained a point mutation D835Y in FLT-3. Samples # 3 and # 4 contained the wild-type FLT-3. The table indicates that in samples # 1 and # 2, co-treatment with LAQ824 and PKC412 resulted in a higher % of non-viable cells than treatment with either agent alone. In contrast, this was not the case in samples # 3 and # 4. Although exposure to LAQ824 increased the % of non-viable cells in a dose-dependent manner, this was clearly less than in samples # 1 and # 2. PKC412 treatment also increased the % of non-viable cells in samples # 1 and # 2 in a dose-dependent manner, while there was none to a minimal increase in the % of non-viable cells in samples # 3 and # 4.

Following co-treatment with LAQ824 and PKC412, Western blot analyses of the total cell lysates of sample # 1 showed a greater decline in the p-FLT-3 and FLT-3 levels than treatment with either agent alone. These data strongly suggest that as compared to either agent alone, the combination of LAQ824 and PKC412 exerts greater cytotoxicity against mutant versus wild-type FLT-3-containing primary AML cells. Since diverse mutations may have different sensitivity to FLT-3 kinase inhibitors, these findings also generate the rationale to investigate the clinical efficacy in AML of the combination that includes not only a FLT-3 kinase inhibitor but also LAQ824 that lowers the levels of mutant FLT-3.

## Claims

1. Use of a combination of (a) a FLT-3 inhibitor and (b) a histone deacetylase inhibitor (HDAI) for the preparation of a medicament for the treatment of acute myeloid leukemia (AML), colorectal cancer (CRC) or non-small cell lung cancer (NSCLC), wherein the FLT-3 inhibitor is N-[(9*S*,10*R*,11*R*,13*R*)-2,3,10,11,12,13-hexahydro-10-methoxy-9-methyl-1-oxo-9,13-epoxy-1*H*,9*H*-diindolo[1,2,3-gh:3',2',1'-Im]pyrrolo[3,4-j][1,7]benzodiazonin-11-yl]-*N-*methylbenzamide of the formula (VII): 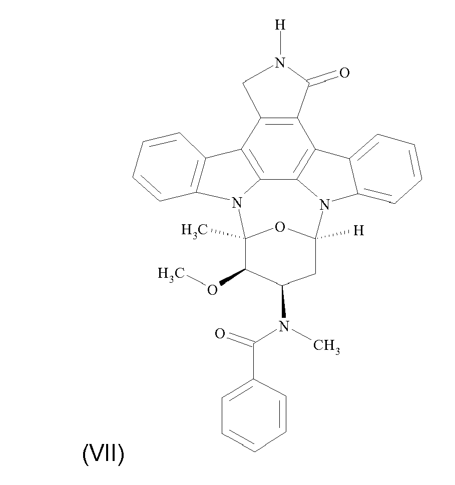 or a salt thereof and the HDAI is *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof.

2. Use of the combination according to claim 1 for simultaneous, concurrent, separate or sequential use.

3. A pharmaceutical composition comprising (a) a FLT-3 inhibitor and (b) a histone deacetylase inhibitor for use in treating of acute myeloid leukemia, wherein the FLT-3 inhibitor is N-[(9*S*,10*R*,11*R*,13*R*)-2,3,10,11,12,13-hexahydro-10-methoxy-9-methyl-1-oxo-9,13-epoxy-1*H*,9*H*-diindolo[1,2,3-gh:3',2',1'-Im]pyrrolo[3,4-j][1,7]benzodiazonin-11-yl]-*N-*methylbenzamide of the formula (VII): 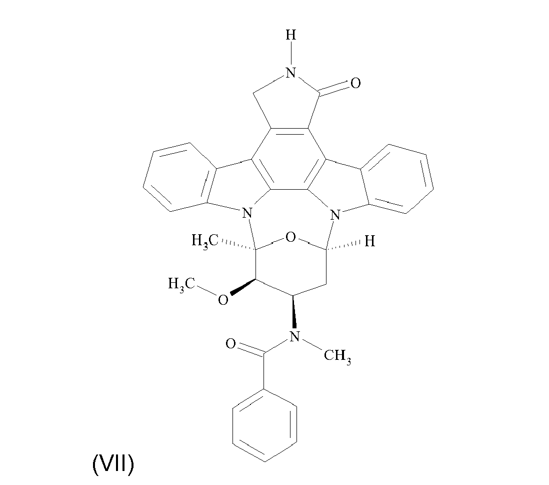 or a salt thereof and the HDAI is *N*-hydroxy-3-[4-[[[2-(2-methyl-1*H*-indol-3-yl)-ethyl]-amino]methyl]phenyl]-2*E*-2-propenamide or a pharmaceutically acceptable salt thereof.

4. The pharmaceutical composition according to claim 3 for simultaneous, concurrent, separate or sequential use.
